# EUROPÄISCHE PATENTANMELDUNG

(11) **EP 1 754 715 A1**
(43) Veröffentlichungstag der Anmeldung: **21.02.2007**
(21) Anmeldenummer: 05090240.2
(22) Anmeldetag: 19.08.2005
(51) Int. Cl.: C07K 14/16, C07K 16/10, C07K 19/00, C12N 15/48, C12N 15/62, A61K 39/21, G01N 33/68

(54) **Impfstoff auf Basis virusneutralisierender Antikörper**

(71) Anmelder: Bundesrepublik Deutschland vertreten durch das Bundesminsterium für Gesundheit, dieses vertr. durch das Robert-Koch-Institut, 13353 Berlin (DE)
(72) Erfinder: Denner, Joachim, Dr., 10625 Berlin (DE); Langhammer, Stefan, 10961 Berlin (DE); Fiebig, Uwe, 14473 Potsdam (DE); Kurth, Reinhard, Prof. Dr., 14532 Kleinmachnow (DE)
(74) Vertreter: Ziebig, Marlene

(57) **Zusammenfassung**

Die Erfindung betrifft immunogene Konstrukte und ein pharmazeutisches Mittel zur Induktion von humoralen neutralisierenden Immunantworten gegen Retrovirusinfektionen, vorrangig HIV. Die Erfindung betrifft weiterhin ein Verfahren zur Induktion einer Antikörperantwort und ein Verfahren zur passiven Immunisierung eines Organismus unter Verwendung neutralisierender Antikörper, die mit den genannten immunogenen Konstrukten gewonnen wurden.

SEQUENZPROTOKOLL

<110> BRD, vertreten durch das Robert-Koch-Institut

<120> Impfstoff auf Basis virusneutralisierender Antikörper

<130> P347805EP

<140>
<141>

<160> 90

<170> PatentIn Ver. 2.1

<210> 1
<211> 13
<212> PRT
<213> Human immunodeficiency virus type 1

<220>
<221> SIMILAR
<222> (1)..(13)
<223> gp41 E1

<400> 1
Gln Ala Arg Gln Leu Leu Ser Asp Ile Val Gln Gln Gln
 1               5                   10

<210> 2
<211> 13
<212> PRT
<213> Human immunodeficiency virus type 1

<220>
<221> SIMILAR
<222> (1)..(13)
<223> gp41 E1

<400> 2
Glu Leu Asp Lys Trp Ala Ser Leu Trp Asn Trp Phe Asn
 1               5                   10

<210> 3
<211> 21
<212> PRT

<213> Human immunodeficiency virus type 1

<220>
<221> SIMILAR
<222> (1)..(21)
<223> gp41 E1

<400> 3
Gly Ala Ser Val Thr Leu Thr Val Gln Ala Arg Gln Leu Leu Ser Asp
1               5                   10                  15

Ile Val Gln Gln Gln
            20

<210> 4
<211> 20
<212> PRT
<213> Human immunodeficiency virus type 1

<220>
<221> SIMILAR
<222> (1)..(20)
<223> gp41 E1

<400> 4
Glu Leu Asp Lys Trp Ala Ser Leu Trp Asn Trp Phe Asn Ile Thr Asn
1               5                   10                  15

Trp Leu Trp Tyr
            20

<210> 5
<211> 24
<212> PRT
<213> Human immunodeficiency virus type 1

<220>
<221> SIMILAR
<222> (1)..(24)
<223> gp41 E1

<400> 5
Leu Gly Ala Ala Gly Ser Thr Met Gly Ala Ala Ser Val Thr Leu Thr
1               5                   10                  15

Val Gln Ala Arg Leu Leu Leu Ser

            20

<210> 6
<211> 24
<212> PRT
<213> Human immunodeficiency virus type 1

<220>
<221> SIMILAR
<222> (1)..(24)
<223> gp41 E2

<400> 6
Asn Glu Gln Glu Leu Leu Glu Leu Asp Lys Trp Ala Ser Leu Trp Asn
1               5                   10                  15

Trp Phe Asp Ile Thr Asn Trp Leu
            20

<210> 7
<211> 28
<212> PRT
<213> Human immunodeficiency virus type 1

<220>
<221> SIMILAR
<222> (1)..(28)
<223> gp41 E1

<400> 7
Phe Leu Gly Phe Leu Gly Ala Ala Gly Ser Thr Met Gly Ala Ala Ser
1               5                   10                  15

Ile Thr Leu Thr Val Gln Ala Arg Gln Leu Leu Ser
            20                  25

<210> 8
<211> 28
<212> PRT
<213> Human immunodeficiency virus type 1

<220>
<221> SIMILAR
<222> (1)..(28)
<223> gp41 E1

<400> 8
Phe Leu Gly Phe Leu Gly Ala Ala Gly Ser Thr Met Gly Ala Ala Ser
1               5                   10                  15

Met Thr Leu Thr Val Gln Ala Arg Gln Leu Leu Ser
            20                  25

<210> 9
<211> 28
<212> PRT
<213> Human immunodeficiency virus type 1

<220>
<221> SIMILAR
<222> (1)..(28)
<223> gp41 E1

<400> 9
Phe Leu Gly Phe Leu Gly Ala Ala Gly Ser Thr Met Gly Ala Ala Ser
1               5                   10                  15

Leu Thr Leu Thr Val Gln Ala Arg Gln Leu Leu Ser
            20                  25

<210> 10
<211> 28
<212> PRT
<213> Human immunodeficiency virus type 1

<220>
<221> SIMILAR
<222> (1)..(28)
<223> gp41 E1

<400> 10
Leu Leu Gly Phe Leu Gly Ala Ala Gly Ser Thr Met Gly Ala Ala Ser
1               5                   10                  15

Ile Thr Leu Thr Val Gln Ala Arg Gln Leu Leu Ser
            20                  25

<210> 11
<211> 28
<212> PRT

<213> Human immunodeficiency virus type 1

<220>
<221> SIMILAR
<222> (1)..(28)
<223> gp41 E1

<400> 11
Phe Leu Gly Phe Leu Gly Ala Ala Gly Ser Thr Met Gly Ala Ala Ser
1               5                   10                  15

Ile Thr Leu Thr Val Gln Val Arg Gln Leu Leu Ser
            20                  25



<210> 12
<211> 28
<212> PRT
<213> Human immunodeficiency virus type 1

<220>
<221> SIMILAR
<222> (1)..(28)
<223> gp41 E1

<400> 12
Phe Leu Gly Val Leu Ser Ala Ala Gly Ser Thr Met Gly Ala Ala Ala
1               5                   10                  15

Thr Ala Leu Thr Val Gln Thr His Thr Leu Met Lys
            20                  25



<210> 13
<211> 28
<212> PRT
<213> Human immunodeficiency virus type 1

<220>
<221> SIMILAR
<222> (1)..(28)
<223> gp41 E2

<400> 13
Asn Glu Gln Asp Leu Leu Ala Leu Asp Lys Trp Ala Ser Leu Trp Asn
1               5                   10                  15

Trp Phe Asp Ile Thr Asn Trp Leu Trp Tyr Ile Lys

20                  25

<210> 14
<211> 28
<212> PRT
<213> Human immunodeficiency virus type 1

<220>
<221> SIMILAR
<222> (1)..(28)
<223> gp41 E2

<400> 14
Asn Glu Gln Asp Leu Leu Ala Leu Asp Lys Trp Ala Asn Leu Trp Asn
1               5                   10                  15

Trp Phe Asp Ile Ser Asn Trp Leu Trp Tyr Ile Lys
            20                  25

<210> 15
<211> 28
<212> PRT
<213> Human immunodeficiency virus type 1

<220>
<221> SIMILAR
<222> (1)..(28)
<223> gp41 E2

<400> 15
Asn Glu Gln Asp Leu Leu Ala Leu Asp Lys Trp Ala Asn Leu Trp Asn
1               5                   10                  15

Trp Phe Asp Ile Thr Asn Trp Leu Trp Tyr Ile Arg
            20                  25

<210> 16
<211> 28
<212> PRT
<213> Human immunodeficiency virus type 1

<220>
<221> SIMILAR
<222> (1)..(28)
<223> gp41 E2

<400> 16
Asn Glu Gln Glu Leu Leu Glu Leu Asp Lys Trp Ala Ser Leu Trp Asn
1               5                   10                  15

Trp Phe Asp Ile Thr Asn Trp Leu Trp Tyr Ile Lys
            20                  25

<210> 17
<211> 28
<212> PRT
<213> Human immunodeficiency virus type 1

<220>
<221> SIMILAR
<222> (1)..(28)
<223> gp41 E2

<400> 17
Asn Glu Lys Asp Leu Leu Ala Leu Asp Ser Trp Gln Asn Leu Trp Asn
1               5                   10                  15

Trp Phe Asp Ile Thr Asn Trp Leu Trp Tyr Ile Lys
            20                  25

<210> 18
<211> 28
<212> PRT
<213> Human immunodeficiency virus type 1

<220>
<221> SIMILAR
<222> (1)..(28)
<223> gp41 E2

<400> 18
Asn Glu Gln Glu Leu Leu Glu Leu Asp Lys Trp Ala Ser Leu Trp Asn
1               5                   10                  15

Trp Phe Ser Ile Thr Gln Trp Leu Trp Tyr Ile Lys
            20                  25

<210> 19
<211> 28
<212> PRT

<213> Human immunodeficiency virus type 1

<220>
<221> SIMILAR
<222> (1)..(28)
<223> gp41 E2

<400> 19
Asn Glu Gln Glu Leu Leu Ala Leu Asp Lys Trp Ala Ser Leu Trp Asn
1               5                   10                  15

Trp Phe Asp Ile Ser Asn Trp Leu Trp Tyr Ile Lys
            20                  25



<210> 20
<211> 28
<212> PRT
<213> Human immunodeficiency virus type 1

<220>
<221> SIMILAR
<222> (1)..(28)
<223> gp41 E2

<400> 20
Asn Glu Gln Asp Leu Leu Ala Leu Asp Lys Trp Asp Asn Leu Trp Ser
1               5                   10                  15

Trp Phe Ser Ile Thr Asn Trp Leu Trp Tyr Ile Lys
            20                  25



<210> 21
<211> 28
<212> PRT
<213> Human immunodeficiency virus type 1

<220>
<221> SIMILAR
<222> (1)..(28)
<223> gp41 E2

<400> 21
Asn Glu Gln Asp Leu Leu Ala Leu Asp Lys Trp Ala Ser Leu Trp Asn
1               5                   10                  15

Trp Phe Asp Ile Thr Lys Trp Leu Trp Tyr Ile Lys

20                  25

<210> 22
<211> 28
<212> PRT
<213> Human immunodeficiency virus type 1

<220>
<221> SIMILAR
<222> (1)..(28)
<223> gp41 E2

<400> 22
Asn Glu Gln Asp Leu Leu Ala Leu Asp Lys Trp Ala Ser Leu Trp Asn
1               5                   10                  15

Trp Phe Ser Ile Thr Asn Trp Leu Trp Tyr Ile Lys
            20                  25

<210> 23
<211> 28
<212> PRT
<213> Human immunodeficiency virus type 1

<220>
<221> SIMILAR
<222> (1)..(28)
<223> gp41 E2

<400> 23
Asn Glu Lys Lys Leu Leu Glu Leu Asp Glu Trp Ala Ser Ile Trp Asn
1               5                   10                  15

Trp Leu Asp Ile Thr Lys Trp Leu Trp Tyr Ile Lys
            20                  25

<210> 24
<211> 35
<212> PRT
<213> Bovine immunodeficiency virus

<220>
<221> SIMILAR
<222> (1)..(35)
<223> gp40 E1

<400> 24
Ala Val Gly Leu Ala Ile Phe Leu Leu Val Leu Ala Ile Met Ala Ile
1               5                   10                  15

Thr Ser Ser Leu Val Ala Ala Thr Thr Leu Val Asn Gln His Thr Thr
            20                  25                  30

Ala Lys Val
        35

<210> 25
<211> 33
<212> PRT
<213> Bovine immunodeficiency virus

<220>
<221> SIMILAR
<222> (1)..(33)
<223> gp40 E2

<400> 25
Ser Leu Ser Asp Thr Gln Asp Thr Phe Gly Leu Glu Thr Ser Ile Phe
1               5                   10                  15

Asp His Leu Val Gln Leu Phe Asp Trp Thr Ser Trp Lys Asp Trp Ile
            20                  25                  30

Lys

<210> 26
<211> 41
<212> PRT
<213> Caprine arthritis-encephalitis virus

<220>
<221> SIMILAR
<222> (1)..(41)
<223> Hüllprotein E1

<400> 26
Gly Val Gly Leu Val Ile Met Leu Val Ile Met Ala Ile Val Ala Ala
1               5                   10                  15

Ala Gly Ala Ser Leu Gly Val Ala Asn Ala Ile Gln Gln Ser Tyr Thr

            20                  25                  30

Lys Ala Ala Val Gln Thr Leu Ala Asn
        35                  40

<210> 27
<211> 36
<212> PRT
<213> Caprine arthritis-encephalitis virus

<220>
<221> SIMILAR
<222> (1)..(36)
<223> Hüllprotein E2

<400> 27
Ala Met Thr Gln Leu Ala Glu Glu Gln Ala Arg Arg Ile Pro Glu Val
1               5                   10                  15

Trp Glu Ser Leu Lys Asp Val Phe Asp Trp Ser Gly Trp Phe Ser Trp
            20                  25                  30

Leu Lys Tyr Ile
        35

<210> 28
<211> 39
<212> PRT
<213> Equine infectious anemia virus

<220>
<221> SIMILAR
<222> (1)..(39)
<223> gp45 E1

<400> 28
Phe Gly Ile Ser Ala Ile Val Ala Ala Ile Val Ala Ala Thr Ala Ile
1               5                   10                  15

Ala Arg Ser Ala Thr Met Ser Tyr Val Ala Leu Thr Glu Val Asn Lys
            20                  25                  30

Ile Met Glu Val Gln Asn His
        35

<210> 29
<211> 39
<212> PRT
<213> Equine infectious anemia virus

<220>
<221> SIMILAR
<222> (1)..(39)
<223> gp45 E2

<400> 29
Leu Ala Gln Ser Met Ile Thr Phe Asn Thr Pro Asp Ser Ile Ala Gln
1               5                   10                  15

Phe Gly Lys Asp Leu Trp Ser His Ile Gly Asn Trp Ile Pro Gly Leu
            20                  25                  30

Gly Ala Ser Ile Ile Lys Tyr
        35

<210> 30
<211> 48
<212> PRT
<213> Feline immunodeficiency virus

<220>
<221> SIMILAR
<222> (1)..(48)
<223> gp36 E1

<400> 30
Ser Ser Ser Tyr Ser Gly Thr Lys Met Ala Cys Pro Ser Asn Arg Gly
1               5                   10                  15

Ile Leu Arg Asn Trp Tyr Asn Pro Val Ala Gly Leu Arg Gln Ser Leu
            20                  25                  30

Glu Gln Tyr Gln Val Val Lys Gln Pro Asp Tyr Leu Leu Val Pro Glu
        35                  40                  45

<210> 31
<211> 35
<212> PRT

<213> Feline immunodeficiency virus

<220>
<221> SIMILAR
<222> (1)..(35)
<223> gp36 E2

<400> 31
Met Asp Ile Glu Gln Asn Asn Val Gln Gly Lys Ile Gly Ile Gln Gln
1               5                   10                  15

Leu Gln Lys Trp Glu Asp Trp Val Arg Trp Ile Gly Asn Ile Pro Gln
            20                  25                  30

Tyr Leu Lys
        35

<210> 32
<211> 49
<212> PRT
<213> Ovine maedi visna virus

<220>
<221> SIMILAR
<222> (1)..(49)
<223> gp41 E1

<400> 32
Gly Ile Gly Leu Val Ile Val Leu Ala Ile Met Ala Ile Ile Ala Ala
1               5                   10                  15

Ala Gly Ala Gly Leu Gly Val Ala Asn Ala Val Gln Gln Ser Ser Tyr
            20                  25                  30

Thr Arg Thr Ala Val Gln Ser Leu Ala Asn Ala Thr Ala Ala Gln Gln
        35                  40                  45

Asn

<210> 33
<211> 37
<212> PRT
<213> Ovine maedi visna virus

<220>

<221> SIMILAR
<222> (1)..(37)
<223> gp41 E2

<400> 33
Gln Val Gln Ile Ala Gln Arg Asp Ala Gln Arg Ile Pro Asp Val Trp
1               5                   10                  15

Lys Ala Leu Gln Glu Ala Phe Asp Trp Ser Gly Trp Phe Ser Trp Leu
            20                  25                  30

Lys Tyr Ile Pro Trp
        35


<210> 34
<211> 42
<212> PRT
<213> Simian immunodeficiency virus

<220>
<221> SIMILAR
<222> (1)..(42)
<223> gp41 E1

<400> 34
Leu Gly Phe Leu Gly Phe Leu Ala Thr Ala Gly Ser Ala Met Gly Ala
1               5                   10                  15

Ala Ser Leu Val Thr Ala Gln Ser Arg Thr Leu Leu Ala Val Ile Val
            20                  25                  30

Gln Gln Gln Gln Gln Leu Leu Asp Val Val
        35                  40


<210> 35
<211> 40
<212> PRT
<213> Simian immunodeficiency virus

<220>
<221> SIMILAR
<222> (1)..(40)
<223> gp41 E2

<400> 35
Glu Glu Ala Gln Ile Gln Gln Glu Lys Asn Met Tyr Glu Leu Trp Lys

1               5                   10                  15

Leu Asn Trp Trp Asp Val Phe Gly Asn Trp Phe Asp Leu Thr Ser Trp
            20                  25                  30

Asp Leu Thr Ser Trp Ile Lys Tyr
        35                  40

<210> 36
<211> 40
<212> PRT
<213> Simian immunodeficiency virus

<220>
<221> SIMILAR
<222> (1)..(40)
<223> gp41 E1

<400> 36
Leu Gly Ala Leu Gly Phe Leu Gly Ala Ala Gly Ser Thr Met Gly Ala
1               5                   10                  15

Ala Ala Val Thr Leu Thr Val Gln Ala Arg Gln Leu Leu Ser Gly Ile
            20                  25                  30

Val Gln Gln Gln Asn Asn Leu Leu
        35                  40

<210> 37
<211> 37
<212> PRT
<213> Simian immunodeficiency virus

<220>
<221> SIMILAR
<222> (1)..(37)
<223> gp41 E2

<400> 37
Glu Glu Ala Gln Ser Gln Gln Glu Lys Asn Glu Arg Asp Leu Leu Glu
1               5                   10                  15

Leu Asp Gln Trp Ala Ser Leu Trp Asn Trp Phe Asp Ile Thr Lys Trp
            20                  25                  30

Leu Trp Tyr Ile Lys

        35

<210> 38
<211> 48
<212> PRT
<213> Visna virus

<220>
<221> SIMILAR
<222> (1)..(48)
<223> gp41 E1

<400> 38
Gly Ile Gly Leu Val Ile Val Leu Ala Ile Met Ala Ile Ile Ala Ala
1               5                   10                  15

Ala Gly Ala Gly Leu Gly Val Ala Asn Ala Val Gln Gln Ser Tyr Thr
            20                  25                  30

Arg Thr Ala Val Gly Ser Leu Ala Asn Ala Thr Ala Ala Gln Gln Glu
        35                  40                  45

<210> 39
<211> 39
<212> PRT
<213> Visna virus

<220>
<221> SIMILAR
<222> (1)..(39)
<223> gp41 E2

<400> 39
Glu Ala Ala Leu Gln Val His Ile Ala Gln Arg Asp Ala Arg Arg Ile
1               5                   10                  15

Pro Asp Ala Trp Lys Ala Ile Gln Glu Ala Phe Asn Asn Trp Ser Ser
            20                  25                  30

Trp Phe Ser Trp Leu Lys Tyr
        35

<210> 40
<211> 40
<212> PRT
<213> Human immunodeficiency virus type 2

<220>
<221> SIMILAR
<222> (1)..(40)
<223> gp36 E1

<400> 40
Leu Gly Phe Leu Gly Phe Leu Ala Thr Ala Gly Ser Ala Met Gly Ala
1               5                   10                  15

Arg Ser Leu Thr Leu Ser Ala Gln Ser Arg Thr Leu Leu Ala Gly Ile
            20                  25                  30

Val Gln Gln Gln Gln Gln Leu Leu
        35                  40


<210> 41
<211> 36
<212> PRT
<213> Human immunodeficiency virus type 2

<220>
<221> SIMILAR
<222> (1)..(36)
<223> gp36 E2

<400> 41
Glu Glu Ala Gln Ile Gln Glu Lys Asn Met Tyr Glu Leu Gln Lys Leu
1               5                   10                  15

Asn Ser Trp Asp Ile Leu Gly Asn Trp Phe Asp Leu Ile Ser Trp Val
            20                  25                  30

Lys Tyr Ile Gln
        35


<210> 42
<211> 43
<212> PRT
<213> Rous sarcoma virus

<220>

<221> SIMILAR
<222> (1)..(43)
<223> gp37 E1

<400> 42
Trp Gly Pro Thr Ala Arg Ile Phe Ala Ser Ile Leu Ala Pro Gly Val
1               5                   10                  15

Ala Ala Ala Gln Ala Leu Arg Glu Ile Glu Arg Leu Ala Cys Trp Ser
            20                  25                  30

Val Lys Gln Ala Asn Leu Thr Thr Ser Leu Leu
        35                  40


<210> 43
<211> 36
<212> PRT
<213> Rous sarcoma virus

<220>
<221> SIMILAR
<222> (1)..(36)
<223> gp37 E2

<400> 43
Lys Phe Gln Leu Met Lys Lys His Val Asn Lys Ile Gly Val Asp Ser
1               5                   10                  15

Asp Pro Ile Gly Ser Trp Leu Arg Gly Ile Phe Gly Gly Ile Gly Glu
            20                  25                  30

Trp Ala Val His
        35


<210> 44
<211> 42
<212> PRT
<213> Avian leukosis virus

<220>
<221> SIMILAR
<222> (1)..(42)
<223> gp37 E1

<400> 44
Ser Val Ser His Leu Ser Ser Asp Cys Asn Asp Glu Val Gln Leu Trp

1               5                   10                  15

Ser Val Thr Ala Arg Ile Phe Ala Ser Phe Phe Ala Gly Val Ala Ala
            20                  25                  30

Gln Ala Leu Lys Glu Ile Glu Arg Leu Ala
        35                  40



<210> 45
<211> 35
<212> PRT
<213> Avian leukosis virus

<220>
<221> SIMILAR
<222> (1)..(35)
<223> gp37 E2

<400> 45
Ala Leu Gln Ala Met Lys Glu His Thr Glu Lys Ile Arg Val Glu Asp
1               5                   10                  15

Asp Ile Gly Asp Trp Phe Thr Arg Thr Phe Gly Gly Leu Gly Gly Trp
            20                  25                  30

Leu Ala Lys
        35



<210> 46
<211> 49
<212> PRT
<213> Jaagsiekte sheep retrovirus

<220>
<221> SIMILAR
<222> (1)..(49)
<223> gp36 E1

<400> 46
Gly Leu Ser Leu Ile Ile Leu Gly Ile Val Ser Leu Ile Thr Leu Ile
1               5                   10                  15

Ala Thr Ala Val Thr Ala Cys Cys Ser Leu Ala Gln Ser Ile Gln Ala
            20                  25                  30

Ala His Thr Val Asp Leu Ser Ser Gln Asn Val Thr Lys Val Met Gly

35                  40                  45

Thr

<210> 47
<211> 36
<212> PRT
<213> Jaagsiekte sheep retrovirus

<220>
<221> SIMILAR
<222> (1)..(36)
<223> gp36 E2

<400> 47
Ile Glu Asn Ser Pro Lys Ala Thr Leu Asn Ile Ala Asp Thr Val Asp
1               5                   10                  15

Asn Phe Leu Gln Asn Leu Phe Ser Asn Phe Pro Ser Leu His Ser Leu
            20                  25                  30

Asn Lys Thr Leu
        35

<210> 48
<211> 49
<212> PRT
<213> Squirrel monkey retrovirus

<220>
<221> SIMILAR
<222> (1)..(49)
<223> gp20 E1

<400> 48
Ala Val Thr Leu Ile Pro Leu Leu Val Gly Leu Gly Val Ser Thr Ala
1               5                   10                  15

Val Ala Thr Gly Thr Ala Gly Leu Gly Val Ala Val Gln Ser Tyr Thr
            20                  25                  30

Lys Leu Ser His Gln Leu Ile Asn Asp Val Gln Ala Leu Ser Ser Thr
        35                  40                  45

Ile

<210> 49
<211> 34
<212> PRT
<213> Squirrel monkey retrovirus

<220>
<221> SIMILAR
<222> (1)..(34)
<223> gp20 E2

<400> 49
Lys Ile Lys Asn Leu Gln Glu Asp Leu Glu Lys Arg Arg Lys Ala Leu
1               5                   10                  15

Ala Asp Asn Leu Phe Leu Thr Gly Leu Asn Gly Leu Leu Pro Tyr Leu
            20                  25                  30

Leu Pro

<210> 50
<211> 49
<212> PRT
<213> Simian retrovirus

<220>
<221> SIMILAR
<222> (1)..(49)
<223> gp20 E1

<400> 50
Ala Ile Gln Phe Ile Pro Leu Val Ile Gly Leu Gly Ile Thr Thr Ala
1               5                   10                  15

Val Ser Thr Gly Thr Ala Gly Leu Gly Val Ser Leu Thr Trp Tyr Thr
            20                  25                  30

Lys Leu Ser His Gln Leu Ile Ser Asp Asx Gln Ala Ile Ser Ser Thr
        35                  40                  45

Ile

<210> 51
<211> 34
<212> PRT
<213> Simian retrovirus

<220>
<221> SIMILAR
<222> (1)..(34)
<223> gp20 E2

<400> 51
Lys Ile Lys Asn Leu Gln Asp Asp Leu Glu Lys Arg Arg Lys Gln Leu
1               5                   10                  15

Ile Asp Asn Pro Phe Trp Thr Gly Phe His Leu Leu Pro Tyr Val Met
            20                  25                  30

Pro Leu

<210> 52
<211> 40
<212> PRT
<213> Künstliche Sequenz

<220>
<221> SIMILAR
<222> (1)..(40)
<223> Hüllprotein E1

<220>
<223> Beschreibung der künstlichen Sequenz: Peptid aus
      der Epitop-Region E1 eines viralen transmembranen
      Hüllproteins

<400> 52
Asp Pro Val Ser Leu Thr Val Ala Leu Leu Leu Gly Gly Leu Thr Met
1               5                   10                  15

Gly Ser Leu Ala Ala Gly Ile Gly Thr Gly Thr Ala Ala Leu Ile Glu
            20                  25                  30

Thr Asn Gln Phe Lys Gln Leu Gln
        35                  40

<210> 53

<211> 35
<212> PRT
<213> Murine leukemia virus

<220>
<221> SIMILAR
<222> (1)..(35)
<223> p15E E2

<400> 53
Ser Met Ala Lys Leu Arg Glu Arg Phe Lys Gln Arg Gln Lys Leu Phe
1               5                   10                  15

Glu Ser Gln Gln Gly Gln Phe Glu Gly Trp Tyr Asn Lys Ser Pro Trp
            20                  25                  30

Glu Thr Thr
        35



<210> 54
<211> 45
<212> PRT
<213> Gibbon ape leukemia virus

<220>
<221> SIMILAR
<222> (1)..(45)
<223> p15E E1

<400> 54
Ala Val Ser Leu Thr Leu Ala Val Leu Leu Gly Leu Gly Ile Thr Ala
1               5                   10                  15

Gly Ile Gly Gly Ser Thr Ala Leu Ile Lys Gly Pro Ile Asp Leu Gln
            20                  25                  30

Gln Gly Leu Thr Ser Leu Gln Ile Ala Ile Asp Ala Asp
        35                  40                  45



<210> 55
<211> 35
<212> PRT
<213> Gibbon ape leukemia virus

<220>
<221> SIMILAR

<222> (1)..(35)
<223> p15E E2

<400> 55
Ser Met Lys Lys Leu Lys Glu Lys Leu Asp Lys Arg Gln Leu Glu Arg
1               5                   10                  15

Gln Asp Ser Gln Asn Trp Tyr Glu Gly Trp Phe Asn Asn Trp Pro Trp
            20                  25                  30

Phe Thr Thr
        35

<210> 56
<211> 44
<212> PRT
<213> Murine leukemia virus

<220>
<221> SIMILAR
<222> (1)..(44)
<223> p15E E1

<400> 56
Glu Pro Val Ser Leu Thr Leu Ala Leu Leu Leu Gly Gly Leu Thr Met
1               5                   10                  15

Gly Gly Ile Ala Gly Val Gly Thr Gly Thr Thr Ala Leu Val Ala Thr
            20                  25                  30

Gln Gln Phe Gln Gln Leu Gln Ala Ala Met His Asp
        35                  40

<210> 57
<211> 35
<212> PRT
<213> Murine leukemia virus

<220>
<221> SIMILAR
<222> (1)..(35)
<223> p15E E2

<400> 57
Ser Met Ala Lys Leu Arg Glu Arg Leu Ser Gln Arg Gln Lys Leu Phe
1               5                   10                  15

Glu Ser Gln Gln Gly Trp Phe Glu Gly Leu Phe Asn Lys Ser Pro Trp
            20                  25                  30

Phe Thr Thr
        35

<210> 58
<211> 44
<212> PRT
<213> Feline leukemia virus

<220>
<221> SIMILAR
<222> (1)..(44)
<223> p15E E1

<400> 58
Glu Pro Ile Ser Leu Thr Val Ala Leu Met Leu Gly Leu Thr Val Gly
1               5                   10                  15

Gly Ile Ala Ala Gly Cys Gly Thr Gly Thr Lys Ala Leu Leu Glu Ala
            20                  25                  30

Gln Phe Leu Gln Leu Gln Met Gln Met His Thr Asp
        35                  40

<210> 59
<211> 35
<212> PRT
<213> Feline leukemia virus

<220>
<221> SIMILAR
<222> (1)..(35)
<223> p15E E2

<400> 59
Asn Met Ala Lys Leu Arg Glu Arg Leu Lys Gln Arg Gln Gln Leu Phe
1               5                   10                  15

Asp Ser Gln Gln Gly Trp Phe Glu Gly Trp Phe Asn Arg Ser Pro Trp
            20                  25                  30

Phe Thr Thr
        35

<210> 60
<211> 43
<212> PRT
<213> Bovine leukemia virus

<220>
<221> SIMILAR
<222> (1)..(43)
<223> gp30 E1

<400> 60
Ser Pro Val Ala Ala Leu Thr Leu Gly Leu Ala Leu Ser Val Gly Leu
1               5                   10                  15

Thr Gly Ile Asn Val Ala Val Ser Ala Leu Ser His Gln Arg Leu Thr
            20                  25                  30

Ser Leu Ile His Val Leu Glu Gln Asp Gln Gln
        35                  40


<210> 61
<211> 27
<212> PRT
<213> Bovine leukemia virus

<220>
<221> SIMILAR
<222> (1)..(27)
<223> gp30 E2

<400> 61
Pro Leu Ser Gln Arg Val Ser Thr Asp Trp Gln Trp Pro Trp Asn Trp
1               5                   10                  15

Asp Leu Gly Leu Thr Ala Trp Val Arg Glu Thr
            20                  25


<210> 62
<211> 37
<212> PRT
<213> Human T-cell lymphotropic virus type 1

<220>
<221> SIMILAR

<222> (1)..(37)
<223> gp21 E1

<400> 62
Ala Val Pro Val Ala Trp Leu Val Ser Ala Leu Ala Met Gly Ala Gly
1               5                   10                  15

Val Ala Gly Gly Ile Thr Gly Ser Met Ser Leu Ala Ser Gly Lys Ser
            20                  25                  30

Leu Leu His Glu Val
        35

<210> 63
<211> 34
<212> PRT
<213> Human T-cell lymphotropic virus type 1

<220>
<221> SIMILAR
<222> (1)..(34)
<223> gp21 E2

<400> 63
Pro Ile Leu Gln Glu Arg Pro Pro Leu Glu Asn Arg Val Leu Thr Gly
1               5                   10                  15

Trp Gly Leu Asn Trp Asp Leu Gly Leu Ser Gln Trp Ala Arg Glu Ala
            20                  25                  30

Leu Gln

<210> 64
<211> 39
<212> PRT
<213> Human T-cell lymphotropic virus type 2

<220>
<221> SIMILAR
<222> (1)..(39)
<223> gp30 E1

<400> 64
Ala Val Pro Ile Ala Val Trp Ser Val Ser Ala Leu Ala Ala Gly Thr
1               5                   10                  15

Gly Ile Ala Gly Gly Val Thr Gly Ser Leu Ser Leu Ala Ser Ser Lys
            20                  25                  30

Ser Leu Leu Leu Glu Val Asp
        35

<210> 65
<211> 34
<212> PRT
<213> Human T-cell lymphotropic virus type 2

<220>
<221> SIMILAR
<222> (1)..(34)
<223> gp30 E2

<400> 65
Ser Val Leu Gln Glu Arg Pro Pro Leu Glu Lys Arg Val Ile Thr Gly
1               5                   10                  15

Trp Gly Leu Asn Trp Asp Leu Gly Leu Ser Gln Trp Ala Arg Glu Ala
            20                  25                  30

Leu Gln

<210> 66
<211> 34
<212> PRT
<213> Human immunodeficiency virus type 1

<220>
<221> SIMILAR
<222> (1)..(34)
<223> gp41 E2

<400> 66
Leu Ile Glu Glu Ser Gln Asn Gln Gln Glu Lys Asn Glu Gln Glu Leu
1               5                   10                  15

Leu Glu Leu Asp Lys Trp Ala Ser Leu Trp Asn Trp Phe Asn Ile Thr
            20                  25                  30

Asn Trp

<210> 67
<211> 17
<212> PRT
<213> Human immunodeficiency virus type 1

<220>
<221> SIMILAR
<222> (1)..(17)
<223> gp41 E2

<400> 67
Gln Asn Gln Gln Glu Lys Asn Glu Gln Glu Leu Leu Glu Leu Asp Lys
1               5                   10                  15

Trp

<210> 68
<211> 10
<212> PRT
<213> Human immunodeficiency virus type 1

<220>
<221> SIMILAR
<222> (1)..(10)
<223> gp41 E2

<400> 68
Gln Gln Glu Lys Asn Glu Gln Glu Leu Leu
1               5                   10

<210> 69
<211> 9
<212> PRT
<213> Human immunodeficiency virus type 1

<220>
<221> SIMILAR
<222> (1)..(9)
<223> gp41 E2

<400> 69
Glu Lys Asn Glu Gln Glu Leu Leu Glu
1               5

<210> 70
<211> 9
<212> PRT
<213> Human immunodeficiency virus type 1

<220>
<221> SIMILAR
<222> (1)..(9)
<223> gp41 E2

<400> 70
Gln Glu Leu Leu Glu Leu Asp Lys Trp
1               5

<210> 71
<211> 12
<212> PRT
<213> Human immunodeficiency virus type 1

<220>
<221> SIMILAR
<222> (1)..(12)
<223> gp41 E2

<400> 71
Asp Lys Trp Ala Ser Leu Trp Asn Trp Phe Asn Ile
1               5                   10

<210> 72
<211> 15
<212> PRT
<213> Human immunodeficiency virus type 1

<220>
<221> SIMILAR
<222> (1)..(15)
<223> gp41 E1

<400> 72
Ala Ala Ser Val Thr Leu Thr Val Gln Ala Arg Leu Leu Leu Ser
1               5                   10                  15

<210> 73

<211> 15
<212> PRT
<213> Human immunodeficiency virus type 1

<220>
<221> SIMILAR
<222> (1)..(15)
<223> gp41 E1

<400> 73
Ala Ala Ala Ala Thr Leu Thr Val Gln Ala Arg Leu Leu Leu Ser
1               5                   10                  15

<210> 74
<211> 15
<212> PRT
<213> Human immunodeficiency virus type 1

<220>
<221> SIMILAR
<222> (1)..(15)
<223> gp41 E1

<400> 74
Ala Ala Ser Val Ala Ala Thr Val Gln Ala Arg Leu Leu Leu Ser
1               5                   10                  15

<210> 75
<211> 15
<212> PRT
<213> Human immunodeficiency virus type 1

<220>
<221> SIMILAR
<222> (1)..(15)
<223> gp41 E1

<400> 75
Ala Ala Ser Val Thr Leu Ala Ala Gln Ala Arg Leu Leu Leu Ser
1               5                   10                  15

<210> 76
<211> 15
<212> PRT
<213> Human immunodeficiency virus type 1

<220>
<221> SIMILAR
<222> (1)..(15)
<223> gp41 E1

<400> 76
Ala Ala Ser Val Thr Leu Thr Val Ala Ala Arg Leu Leu Leu Ser
1               5                   10                  15

<210> 77
<211> 15
<212> PRT
<213> Human immunodeficiency virus type 1

<220>
<221> SIMILAR
<222> (1)..(15)
<223> gp41 E1

<400> 77
Ala Ala Ser Val Thr Leu Thr Val Gln Ala Ala Ala Leu Leu Ser
1               5                   10                  15

<210> 78
<211> 34
<212> PRT
<213> Feline leukemia virus

<220>
<221> SIMILAR
<222> (1)..(34)
<223> p15E E1

<400> 78
Lys Ala Leu Leu Glu Ala Gln Phe Arg Leu Gln Leu Gln Met Gln Met
1               5                   10                  15

His Thr Asp Ile Gln Ala Leu Glu Glu Ser Ile Ser Ala Leu Glu Lys
            20                  25                  30

Ser Leu

<210> 79

<211> 75
<212> PRT
<213> Feline leukemia virus

<220>
<221> SIMILAR
<222> (1)..(75)
<223> p15E E1

<400> 79
Leu Glu Thr Ala Gln Phe Arg Gln Leu Gln Met Ala Met His Thr Asp
1               5                   10                  15

Ile Gln Ala Leu Glu Glu Ser Ile Ser Ala Leu Glu Lys Ser Leu Thr
            20                  25                  30

Ser Leu Ser Glu Val Val Leu Gln Asn Arg Arg Gly Leu Asp Ile Leu
        35                  40                  45

Phe Leu Gln Glu Gly Gly Leu Cys Ala Ala Leu Lys Glu Glu Cys Cys
    50                  55                  60

Phe Tyr Ala Asp His Thr Gly Leu Val Arg Asp
65                  70                  75


<210> 80
<211> 45
<212> PRT
<213> Human immunodeficiency virus type 1

<220>
<221> SIMILAR
<222> (1)..(45)
<223> gp41 E1

<400> 80
Phe Leu Gly Phe Leu Gly Ala Ala Gly Ser Thr Met Gly Ala Arg Ser
1               5                   10                  15

Met Thr Leu Thr Val Gln Ala Arg Gln Leu Leu Ser Gly Ile Val Gln
            20                  25                  30

Gln Gln Asn Asn Leu Leu Arg Ala Ile Glu Ala Gln Gln
        35                  40                  45


<210> 81

<211> 45
<212> PRT
<213> Human immunodeficiency virus type 1

<220>
<221> SIMILAR
<222> (1)..(45)
<223> gp41 E1

<400> 81
Phe Leu Gly Ala Ala Gly Ser Thr Met Gly Ala Ala Ser Met Thr Leu
1               5                   10                  15

Thr Val Gln Ala Arg Gln Leu Leu Ser Gly Ile Val Gln Gln Gln Asn
            20                  25                  30

Asn Leu Leu Arg Ala Ile Glu Ala Gln Gln His Leu Leu
        35                  40                  45


<210> 82
<211> 45
<212> PRT
<213> Human immunodeficiency virus type 1

<220>
<221> SIMILAR
<222> (1)..(45)
<223> gp41 E1

<400> 82
Phe Leu Gly Ala Ala Gly Ser Thr Met Gly Ala Ala Ser Val Thr Leu
1               5                   10                  15

Thr Val Gln Ala Arg Leu Leu Leu Ser Gly Ile Val Gln Gln Gln Asn
            20                  25                  30

Asn Leu Leu Arg Ala Ile Glu Ala Gln Gln His Met Leu
        35                  40                  45


<210> 83
<211> 33
<212> PRT
<213> Human immunodeficiency virus type 1

<220>
<221> SIMILAR

<222> (1)..(33)
<223> gp41 E2

<400> 83
Ser Gln Asn Gln Gln Glu Lys Asn Glu Gln Glu Leu Leu Glu Leu Asp
1               5                   10                  15

Lys Trp Ala Gly Leu Trp Ser Trp Phe Ser Ile Thr Asn Trp Leu Trp
            20                  25                  30

Tyr

<210> 84
<211> 33
<212> PRT
<213> Human immunodeficiency virus type 1

<220>
<221> SIMILAR
<222> (1)..(33)
<223> gp41 E2

<400> 84
Ser Gln Asn Gln Gln Glu Lys Asn Glu Gln Glu Leu Leu Glu Leu Asp
1               5                   10                  15

Lys Trp Ala Ser Leu Trp Asn Trp Phe Asn Ile Thr Asn Trp Leu Trp
            20                  25                  30

Tyr

<210> 85
<211> 33
<212> PRT
<213> Human immunodeficiency virus type 1

<220>
<221> SIMILAR
<222> (1)..(33)
<223> gp41 E2

<400> 85
Ser Gln Thr Gln Gln Glu Lys Asn Glu Gln Glu Leu Leu Glu Leu Asp
1               5                   10                  15

Lys Trp Ala Ser Leu Trp Asn Trp Phe Asp Ile Thr Asn Trp Leu Trp
            20                  25                  30

Tyr

<210> 86
<211> 35
<212> PRT
<213> Koala retrovirus

<220>
<221> SIMILAR
<222> (1)..(35)
<223> p15E E2

<400> 86
Ser Met Arg Arg Leu Lys Glu Arg Leu Asp Lys Arg Gln Leu Glu His
1               5                   10                  15

Gln Lys Asn Leu Ser Trp Tyr Glu Gly Trp Phe Asn Arg Ser Pro Trp
            20                  25                  30

Leu Thr Thr
        35

<210> 87
<211> 36
<212> PRT
<213> Porcine endogenous retrovirus

<220>
<221> SIMILAR
<222> (1)..(36)
<223> p15E E1

<400> 87
Thr Ala Ala Leu Ile Thr Gly Pro Gln Gln Leu Glu Lys Gly Leu Ser
1               5                   10                  15

Asn Leu His Arg Ile Val Thr Glu Asp Leu Gln Ala Leu Glu Lys Ser
            20                  25                  30

Val Ser Asn Leu
        35

<210> 88
<211> 37
<212> PRT
<213> Porcine endogenous retrovirus

<220>
<221> SIMILAR
<222> (1)..(37)
<223> p15E E2

<400> 88
Asp His Ser Gly Ala Ile Arg Asp Ser Met Ser Lys Leu Arg Glu Arg
1               5                   10                  15

Leu Glu Arg Arg Arg Arg Glu Arg Glu Ala Asp Gln Gly Trp Phe Glu
            20                  25                  30

Gly Trp Phe Asn Arg
        35

<210> 89
<211> 37
<212> PRT
<213> Koala retrovirus

<220>
<221> SIMILAR
<222> (1)..(37)
<223> p15E E1

<400> 89
Thr Ala Leu Ile Lys Gly Pro Ile Asp Leu Gln Gln Gly Leu Thr Ser
1               5                   10                  15

Leu Gln Ile Ala Met Asp Thr Asp Leu Arg Ala Leu Gln Asp Ser Ile
            20                  25                  30

Ser Lys Leu Glu Asp
        35

<210> 90
<211> 7
<212> PRT
<213> Feline leukemia virus

<220>
<221> SIMILAR
<222> (1)..(7)
<223> p15E NHR

<400> 90
Ala Leu Glu Glu Ser Ile Ser
1               5

## Beschreibung

Die Erfindung betrifft immunogene Konstrukte und ein pharmazeutisches Mittel zur Induktion von humoralen neutralisierenden Immunantworten gegen Retrovirusinfektionen. Die Erfindung betrifft weiterhin ein Verfahren zur Induktion einer Antikörperantwort und ein Verfahren zur passiven Immunisierung eines Organismus unter Verwendung neutralisierender Antikörper, die mit den genannten immunogenen Konstrukten gewonnen wurden.

Die Infektion mit dem menschlichen Immundefizienzvirus HIV (human immune deficiency virus) und das daraus resultierende erworbene menschliche Immundefizienzsyndrom (AIDS: acquired immune deficiency syndrome) hat sich seit seiner Erstbeschreibung in den frühen achtziger Jahren des letzten Jahrhunderts zu einer der größten globalen Virus-Epidemien entwickelt. 2002 infizierten sich weltweit fünf Millionen Menschen mit HIV, davon 800.000 Kinder unter 15 Jahren, 3,1 Millionen Menschen starben in Folge der HIV-Infektion und AIDS. Die Anzahl der HIV-Infizierten beläuft sich insgesamt auf über 42 Millionen; mehr als 20 Millionen Menschen sind bisher an HIV/AIDS gestorben. Schätzungen gehen davon aus, dass ohne effektive Gegenmaßnahmen im Jahr 2010 45 Millionen Neuinfektionen zu erwarten sind und im Jahr 2020 etwa 70 Millionen Tote.

Im Stand der Technik sind mehrere Therapien von HIV-Infektionen bzw. AIDS bekannt, die jedoch nicht das HI-Virus eliminieren. Stattdessen führen die verwendeten Therapeutika in vielen Fällen zu Resistenzen und müssen als Kombinationstherapie dauerhaft angewandt werden. Bestrebungen gehen deshalb in Richtung einer aktiven Immunisierung und der Induktion neutralisierender Antikörper, wodurch die Infektion und die Integration des viralen Genoms in das zelluläre Genom verhindert werden sollen.

Aus DE 694 26 725 T2 ist bekannt, dass der monoklonale Antikörper 2F5 die hochkonservierte Domäne ELDKWA im Hüllprotein gp41 erkennt und ein breites neutralisierendes Spektrum gegenüber HIV-Stämmen aufweist. Dem Rechnung tragend wurde die Induktion neutralisierender Antikörper durch Verabreichung dieses Epitops in Form eines Peptides bzw. eines modifizierten Peptides in einen Organismus vorgeschlagen. Während DE 694 26 725 T2 die Immunisierung mit unzähligen Formen und Modifikationen des Epitops beschreibt, geht aus der dazugehörigen Veröffentlichung (Muster et al., J Virology 68, 4031-4034 (1994)) hervor, dass Antikörper mit neutralisierender Aktivität gegen HIV lediglich mit einem Konstrukt erzeugt werden, das die o.g. Domäne in der Schleife der Antigenerkennungsstelle B des Influenzavirus HA-Proteins beinhaltet. Jedoch gewährleisten die erzielten moderaten Neutralisationstiter keinen kompletten Infektionsschutz. Hierfür sind Antikörperkonzentrationen, die um 1 bis 2 Zehnerpotenzen über dem 90 %-igen Neutralisationstiter liegen müssen, erforderlich, so dass ein vollständiger in-vivo Schutz bisher fehlt (Parren et al., J. Virology 72, 10270-10274 (1998)). Das Fehlen nachfolgender Publikationen im abgelaufenen Zeitrahmen bestätigt, daß dieser Ansatz nicht erfolgreich war. Die nach der Immunisierung mit einem anderen rekombinanten Fusionsprotein aus der o.g. Domäne und dem Hepatitis B-Virus-Oberflächenantigen (HBsAg) erhaltenen Antiseren konnten HIV-1 in-vitro nicht neutralisieren (Eckhart et al. J Gen Virol. 77, 2001-2008 (1996)).

Es ist des weiteren aus DE 103 39 966 A1 bekannt, dass für die Induktion neutralisierender Antikörper neben der o.g. Domäne ein zweiter Bereich desselben viralen transmembranen Hüllproteins erforderlich ist, der zwischen der Fusionsdomäne und einer alpha-helikalen Struktur des Hüllproteins lokalisiert ist. Durch das Zusammenwirken der beiden Bereiche soll zumindest das die ELDKWA-Sequenz enthaltende Epitop dem Immunsystem so effektiv präsentiert werden, dass neutralisierende Antikörper gegen Viren generiert werden. Unklar ist jedoch, ob der zweite Bereich ein Epitop darstellt oder ausschließlich der Generierung einer höheren antigenen Struktur dient. Ein effektives immunogenes Konstrukt, das auch einen einfachen Aufbau und gute physikochemische Eigenschaften aufweist, wurde bisher nicht beschrieben. Insbesondere ist es bisher notwendig, sämtliche ausgewählte Bereiche aus einem Hüllprotein eines Virus mit aufwendigen molekularbiologischen Techniken in ein anderes Hüllprotein eines anderen Virus einzubringen.

Aufgabe der vorliegenden Erfindung war es deshalb, einen Impfstoff zur Verfügung zu stellen, dessen einfacher Aufbau und hohe Wirksamkeit einen Einsatz in der Prävention und Therapie von retroviralen Erkrankungen gestattet.

Die Aufgabe der Erfindung wird gemäß den unabhängigen Ansprüchen gelöst. Die Unteransprüche beinhalten bevorzugte Ausführungsformen. Erfindungsgemäß wird ein immunogenes Konstrukt bereitgestellt, das Aminosäuresequenzen aus zwei retroviralen transmembranen Hüllproteinen umfasst, dadurch gekennzeichnet, dass das immunogene Konstrukt ein Hüllprotein eines ersten Retrovirus oder einen Teil davon umfasst, wobei das Hüllprotein oder dessen Teil in einem Bereich zwischen Fusionsdomäne und N-terminaler helikaler Region (NHR) eine erste Aminosäuresequenz umfasst, und in einem Bereich zwischen Membrandurchgang und C-terminaler helikaler Region (CHR) eine zweite Aminosäuresequenz beinhaltet, wobei die zweite Aminosäuresequenz eine Sequenz aus einem Hüllprotein eines zweiten Retrovirus ist und diese zweite Aminosäuresequenz die zu ihr in der Sekundär- und/oder Tertiärstruktur homologe zweite Aminosäuresequenz des Hüllproteins des ersten Retrovirus ersetzt, wobei die erste und die zweite Aminosäuresequenz des Konstruktes zueinander antiparallel angeordnet sind. Ihre Interaktion führt zur Bildung neutralisierender Antikörper.

Geeignet ist auch ein Konstrukt, das die die jeweilige Aminosäuresequenz codierende DNA umfasst. Sowohl bei der Auswahl der Aminosäuresequenzen als auch der DNA-Sequenzen ist die Kombination beider Bereiche für die Immunogenität des Konstrukts entscheidend.

Überraschenderweise hat sich gezeigt, dass ein immunogenes Konstrukt, welches mindestens zwei Aminosäuresequenzen aus unterschiedlichen Hüllproteinen verschiedener Retroviren umfasst, in der Lage ist, in einem Organismus neutralisierende Antikörper gegen Retroviren zu induzieren. Unter der Neutralisation eines Retrovirus wird jeder Mechanismus verstanden, der Retroviren in-vivo oder in-vitro an der Infektion hindert und ihre Vermehrung im präventiven Sinne verhindert bzw. im therapeutischen Sinne die weitere Vermehrung der Retroviren hemmt oder eine Kombinationstherapie effektiver werden lässt. Das bedeutet, dass das Immunsystem durch das erfindungsgemäße Konstrukt aktiviert wird.

Die neutralisierenden Antikörper sind vorteilhafterweise gegen die viralen Strukturen gerichtet, durch die sie induziert werden. Es wurde unerwartet gefunden, dass die induzierten neutralisierenden Antikörper insbesondere gegen den Bereich eines Hüllproteins eines Retrovirus, der zwischen Membrandurchgang und C-terminaler helikaler Region (CHR) lokalisiert ist, gerichtet sind. In der vorliegenden Erfindung ist hierunter der zweite Bereich eines Hüllproteins eines zweiten Retrovirus zu verstehen. Während durch die Auswahl einer zweiten Aminosäuresequenz aus dem zweiten Bereich die zu diagnostizierende und/oder zu therapierende Erkrankung determiniert wird, wird offensichtlich durch die erste Aminosäuresequenz und deren erfindungsgemäße antiparallele Anordnung zur zweiten Sequenz diese zweite Sequenz konformationsgerecht präsentiert und dadurch neutralisierende Antikörper gewonnen.

Die Kombination der zweiten Aminosäuresequenz mit einem Hüllproteins aus einem ersten Retrovirus gestaltet sich frei, wenn es über virale Artgrenzen hinweg ein beliebiges retrovirales transmembranes Hüllprotein sein kann, dass die Aufgabe der Erfindung löst. Das Hüllprotein des ersten Retrovirus kann sowohl vollständig oder teilweise in das immunogene Konstrukt der Erfindung Eingang finden. Essentiell ist, dass das erste Hüllprotein oder ein Teil davon in einem Bereich zwischen Fusionsdomäne und N-terminaler helikaler Region (NHR) eine erste Aminosäuresequenz umfasst. Bevorzugt wird die erste Aminosäuresequenz in der NHR von einem Gammaretrovirus und die zweite Aminosäuresequenz in der CHR von einem Lentivirus, vorzugsweise HIV, stammen.

In das Hüllprotein des ersten Retrovirus wird die zweite Aminosäuresequenz des Hüllproteins des zweiten Retrovirus derart eingebracht, dass sie die zweite Aminosäuresequenz des Hüllproteins des ersten Retrovirus, die ebenfalls zwischen Membrandurchgang und C-terminaler helikaler Region (CHR) lokalisiert ist, ersetzt. Ersetzt wird hierbei zunächst die Funktion der zweiten Aminosäuresequenz, wobei im Sinne der Erfindung unter "Funktion der zweiten Aminosäuresequenz" die hervorgerufene Immunantwort, d.h. die Art neutralisierender Antikörper, zu verstehen ist. Darüber hinaus kann auch die Primärstruktur ersetzt werden, indem die ursprüngliche zweite Aminosäuresequenz des Hüllproteins des ersten Virus komplett oder teilweise entfernt wird, vorzugsweise komplett. Bevorzugt ist das Einbringen bzw. Ersetzen durch Substitution. Das erste Hüllprotein kann aber auch als Fragment bereitgestellt werden, in dem die zweite Aminosäuresequenz inhärent deletiert ist, so dass die zweite Aminosäuresequenz des Hüllproteins des zweiten Retrovirus durch Insertion oder Addition eingebracht wird. Natürlich ist es ebenfalls möglich, dass die zweite Aminosäuresequenz des Hüllproteins des zweiten Retrovirus in die zweite Aminosäuresequenz des Hüllproteins des ersten Retrovirus eingefügt wird.

Die zweiten Aminosäuresequenzen der Hüllproteine des ersten und zweiten Retrovirus weisen eine Homologie hinsichtlich ihrer Sekundärstruktur und/oder Tertiärstruktur auf, vorzugsweise der Sekundärstruktur. Dadurch wird eine Zuordnung der zweiten Sequenzen und das gezielte Einbringen an einer definierten Position möglich, in dessen Ergebnis die erste und die zweite Aminosäuresequenz des Konstruktes zueinander antiparallel angeordnet sind. Trotz unterschiedlicher Primärsequenzen des ersten Bereichs in verschiedenen retroviralen Hüllproteinen ist erstaunlicherweise die Funktion konserviert, die in der strukturellen Präsentation von Aminosäuren des zweiten Bereichs zu sehen ist. Ob diese konservierte Funktion auf einer konservierten Tertiärstruktur, die entweder inhärent oder infolge eines "induced fit" vorhanden ist, oder auf einer unspezifischen Stabilisierung beruht, ist spekulativ. Erst die Assoziation der ersten und der zweiten Aminosäuresequenz bedingt jedoch solch eine Konformation zumindest der zweiten Aminosäuresequenz und der darin enthaltenden Epitop-Region, dass nicht nur bindende Antikörper, sondern auch neutralisierende Antikörper erzeugt werden. Die Aufhebung der Beschränkung, nach der Aminosäuresequenzen aus dem Hüllprotein desselben Virus auszuwählen waren, ermöglicht das Bereitstellen jener zweiten Aminosäuresequenz, die für eine spezielle Applikation besonders geeignet ist und/oder vorteilhaft mit einer ersten Aminosäuresequenz interagiert, um die erfindungsgemäße Aufgabe zu lösen.

Diese besondere Eignung bzw. vorteilhafte Interaktion ist dadurch charakterisiert, dass die Induktion von Antikörpern deutlich verstärkt ist, die Bindungseigenschaften verbessert sind und die Antikörper qualitativ und/oder quantitativ das spezifische Retrovirus effektiver neutralisieren. Insbesondere bewirken die erfindungsgemäßen immunogenen Konstrukte das Erreichen hoher Antikörpertiter innerhalb kürzester Immunisierungsperioden, die zu einer effektiven Verringerung der Zahl des Retrovirus, von dem die zweite, C-terminale Aminosäuresequenz stammt, insbesondere von HIV, führen. Die direkte Interaktion der neutralisierenden Antikörper mit dem retroviralen transmembranen Hüllprotein verhindert hierbei die Virusinfektion. Ebenfalls zeichnen sich die erfindungsgemäßen Konstrukte durch eine verbesserte Handhabung aus, die durch vorteilhafte physikochemische Parameter, wie z.B. eine hohe Expressionsrate, Löslichkeit, Stabilität und/oder geringe Größe, möglich wird. Nicht zuletzt können die Material- und Herstellungskosten des immunogenen Konstrukts durch die Auswahl respektive Entwicklung einer Konsensussequenz, die den universellen Einsatz des ersten Bereichs bzw. einer Aminosäuresequenz daraus bei verschiedenen Erkrankungen gestattet, vorteilhaft gesenkt werden.

Erfindungsgemäß hat der Fachmann Aminosäuresequenzen aus mindestens zwei retroviralen transmembranen Hüllproteinen auszuwählen, vorzugsweise zwei Aminosäuresequenzen aus zwei Hüllproteinen. Jedes Hüllprotein hat die Voraussetzung zu erfüllen, dass es über mindestens einen Membrandurchgang mit der Membran assoziiert ist und dass es mindestens eine Fusionsdomäne und mindestens zwei alpha-helikale Strukturen (NHR und CHR) aufweist. Dem Fachmann sind eine Vielzahl an Hüllproteinen bekannt, die diese Voraussetzungen erfüllen. Die Aminosäuresequenzen stammen bevorzugt aus dem zumindest teil- und zeitweise nach außen aus der Membran herausragenden Teil. Die Auswahl konzentriert sich auf zwei definierte Bereiche der Hüllproteine, die beide berücksichtigt werden müssen.

Aus dem Bereich zwischen Membrandurchgang und C-terminaler Helix-Region, wobei diese partiell mitzählt, ist mindestens eine sogenannte zweite Aminosäuresequenz frei auszuwählen, vorzugsweise genau eine zweite Aminosäuresequenz. Die C-terminale Helix-Region ist eine alpha-helikale Struktur, die auch dadurch charakterisiert ist, dass sie der Membran zugewandt (Membran-proximal) ist. Der Wortlaut "der Membran zugewandte alpha-helikale Struktur" beschreibt hierbei nicht die Ausrichtung dieser alpha-helikalen Struktur, sondern lediglich das Verhältnis der mindestens zwei alpha-helikalen Strukturen zur Membran. Die der Membran zugewandte alpha-helikale Struktur besitzt eine geringere räumliche Distanz zur Membran, sofern das Hüllprotein als lineare nicht gefaltete Struktur dargestellt oder angenommen wird. Dem entsprechend liegt die zugewandte Struktur räumlich näher am Membrandurchgang des linear gedachten Hüllproteins. Selbstverständlich ist es möglich, dass durch natürliche oder artifizielle Faltungsvorgänge die Position einzelner Bestandteile des Hüllproteins geändert wird.

Das erste und das zweite Retrovirus bzw. deren Hüllproteine sind frei kombinierbar. So kann die erste, N-terminale Sequenz von einem Hüllprotein eines Gammaretrovirus, wie z.B. FeLV, und die zweite, C-terminale Sequenz von einem Hüllprotein eines Lentivirus, wie z.B. HIV, stammen. Es können auch Kombinationen zweier Gammaretroviren und zweier Lentiviren gewählt werden. Die erste Aminosäuresequenz, die im Bereich zwischen Fusionsdomäne und N-terminaler Helix-Region des Hüllproteins des ersten Virus vorhanden ist, wird durch die zweite Aminosäuresequenz determiniert, wenn die zweite Aminosäuresequenz in den Kontext des Hüllprotein des ersten Retrovirus einbebracht wird und im Ergebnis dessen mit der ersten Aminosäuresequenz interagiert. Die N-terminale Helix-Region kann partiell in die zweite Aminosäuresequenz eingeschlossen sein.

Im Sinne der Erfindung bedeutet die Lage einer ersten Sequenz bzw. das Auswählen einer zweiten Sequenz, die jeweils "zwischen" etwas lokalisiert sind, dass flankierende Segmente, wie z.B. Membrandurchgang, alpha-helikale Strukturen und/oder Fusionsdomäne zumindest teilweise Bestandteile der Sequenzen und damit des immunogenen Konstruktes sein können. Die Kombination der Aminosäuresequenzen aus dem ersten und zweiten Bereich führt zur Induktion der neutralisierenden Antikörper. Die erste (N-terminale) und zweite (C-terminale) Aminosäuresequenz können deshalb auch als Epitop-Region E1 und E2 bezeichnet werden.

Das erfindungsgemäße immunogene Konstrukt wird hergestellt, indem eine erste Aminosäure, vorzugsweise im Kontext eines Hüllproteins, und eine zweite Aminosäuresequenz bereitgestellt und assoziiert werden. Es ist im Sinne der Erfindung möglich, ausgewählte Aminosäuresequenzen, wie z.B. Peptide, Proteindomänen oder Fragmente hiervon, auf verschiedene Weise dem Gesamtprotein zu entnehmen. Natürlich können auch synthetische Peptide oder rekombinante Proteine hergestellt werden, die Aminosäuresequenzen der beiden Bereiche umfassen. Die erhaltenen Peptide, rekombinanten oder viralen Proteine werden als immunogenes Konstrukt einem Organismus appliziert. Alternativ kann die DNA, die die Aminosäuresequenzen codiert, mit gängigen Verfahren erhalten, amplifiziert, verändert oder synthetisiert werden. Vorzugsweise wird die RNA des ersten Hüllproteins des ersten Retrovirus isoliert, in DNA umgeschrieben, ggf. kloniert und die DNA des Hüllproteins oder eines Teils davon amplifiziert. Parallel dazu wird die RNA des zweiten Hüllproteins des zweiten Retrovirus isoliert, in DNA umgeschrieben, ggf. kloniert und die die zweite Aminosäuresequenz codierende DNA amplifiziert. Die beiden erhaltenen DNA-Abschnitte werden ligiert. Anschließend kann die DNA beispielsweise in einen Vektor verpackt und in Zellen transkribiert und translatiert werden.

Grundsätzlich können die immunogenen Konstrukte der vorliegenden Erfindung aus allen Retroviren gewonnen werden, die eine Membran aufweisen, mit der Hüllproteine bzw. ähnliche Strukturen assoziiert sind. Bei der Membran der Retroviren kann es sich um jede Struktur, die Lipide umfasst, handeln, sofern diese es ermöglicht, mit Proteinen eine Verbindung einzugehen. Bevorzugte Retroviren im Sinne der Erfindung sind umhüllte Gammaretroviren. In einer Ausführungsform der Erfindung sind die Retroviren aus der Gruppe von HIV-1, FeLV, BIV, CAEV, EIAV1, FIV, OMVV, SIVmac, SIVcpz, VILV, HIV, HIV-2, RSV, ALV, JSRV, SRV, GALV, MuLV, BLV, HTLV-1, HTLV-2, KoRV, Marburg-Virus und Ebola-Virus ausgewählt. Die Retroviren sind wie folgt abgekürzt:
- BIV: Bovines Immundefizienzvirus
- CAEV: Caprines Arthritis Enzephalitis Virus
- EIAV1: Equines infektiöses Anämievirus
- FIV: Felines Immundefizienzvirus
- OMVV: Ovines Maedi-Visna Virus
- SIVmac: Simianes Immundefizienzvirus aus Makaken
- SIVcpz: Simianes Immundefizienzvirus aus Schimpansen
- VILV: Visna-Lentivirus
- HIV-1: Humanes Immundefizienzvirus Typ 1
- HIV-2: Humanes Immundefizienzvirus Typ 2
- RSV: Rous Sarkom Virus
- ALV: Aviäres Leukosevirus
- JSRV: Jaagsiekte Schaf Retrovirus
- SMRV: Squirrel Monkey Retrovirus
- SRV: Simianes Retrovirus
- GALV: Gibbonaffen Leukämievirus
- MuLV: Murines Leukämievirus
- FeLV: Felines Leukämievirus
- KoRV: Koala Retrovirus
- PERV: Porcines endogenes Retrovirus
- BLV: Bovines Leukämievirus
- HTLV-1: Humanes T-Zell-Leukämievirus Typ 1
- HTLV-2: Humanes T-Zell-Leukämievirus Typ 2

Alle gewonnenen Aminosäuresequenzen, Peptide, rekombinanten Proteine oder retroviralen Proteine können als immunogenes Konstrukt verwendet werden, um neutralisierende Antikörper gegen jenes Retrovirus zu generieren, aus dem die zweite Aminosäuresequenz abgeleitet ist, wobei das Auftreten von Kreuzreaktivitäten sehr unwahrscheinlich ist. Ein immunogenes Konstrukt beispielsweise, dessen erste Aminosäuresequenz aus einem FeLV-Hüllprotein stammt und dessen zweite Aminosäuresequenz aus einem HIV-Hüllprotein ausgewählt ist, wird folglich nur eine Wirkung gegen HIV zeigen. Darüber hinaus ist allerdings aufgrund der Konservierung des zweiten Bereichs innerhalb homologer Hüllproteine eng verwandter Viren (wie z.B. PERV und FeLV) eine Kreuzreaktivität möglich, die sich im gegebenen Beispiel gegen PERV und FeLV richten könnte. Angestrebt ist jedoch, dass die durch das immunogene Konstrukt induzierten neutralisierenden Antikörper ausschließlich gegen das Retrovirus gerichtet sind, aus denen die zweite Aminosäuresequenz gewonnen wird.

In einer bevorzugten Ausführungsform der Erfindung ist das erste Retrovirus ein Gammaretrovirus. In einer besonders bevorzugten Ausführungsform der Erfindung ist das erste Retrovirus FeLV oder PERV. Deren transmembranes Hüllprotein p15E ist ausgesprochen gut löslich.

In einer weiteren bevorzugten Ausführungsform der vorliegenden Erfindung ist das zweite Retrovirus ein Lentivirus. In einer besonders bevorzugten Ausführungsform der Erfindung ist das erste Retrovirus HIV-1.

In einem weiteren Aspekt der vorliegenden Erfindung sind die Hüllproteine aus der Gruppe gp41, p15E, GP, gp20, gp21, gp30, gp36, gp37, gp40, gp45 und gp160 ausgewählt, wobei der Impfstoff eine Kombination von zumindest zwei unterschiedlichen Fragmenten aus mindestens zwei unterschiedlichen Hüllproteinen ist. Die Auswahl von zwei Hüllproteinen kann auch zwei artspezifische homologe, aber nicht identische Varianten desselben Hüllproteins beinhalten. Von allen genannten und beanspruchten Hüllproteinen liegen bereits Sekundär- oder Tertiärstrukturdaten vor, so dass der Fachmann die genannten Bereiche, in denen die erste und zweite Aminosäuresequenz lokalisiert sind, genau bestimmen kann. Erfindungswesentlich ist, dass das immunogene Konstrukt zwei Aminosäuresequenzen aus ganz bestimmten Bereichen von retroviralen transmembranen Hüllproteinen umfasst.

In einer bevorzugten Ausführungsform der Erfindung ist das Hüllprotein des zweiten Retrovirus das transmembrane Hüllprotein eines Lentivirus, vorzugsweise gp41 oder gp36, besonders bevorzugt HIV-1 gp41 oder HIV-2 gp36, ganz besonders bevorzugt HIV-1 gp41. Diese transmembranen Hüllproteine umfassen eine Ectodomäne, einen Anker und einen cytoplasmatischen Teil, wobei die Ectodomäne eine Fusionsdomäne, eine alpha-helikale Struktur, eine Cystein-Cystein-Schleife und eine weitere alpha-helikale Struktur aufweist. Es sind zwei alpha-helikale Strukturen vorhanden, NHR und CHR. C-terminal folgt nach der zweiten alpha-helikalen Struktur eine Ankerdomäne, die das transmembrane Hüllprotein gp41 in der Virusmembran verankert. Das Hüllprotein besitzt hochkonservierte Domänen, die aufgrund der wichtigen Rolle beim Virus-Eintritt nicht mutieren.

Das Hüllprotein des ersten Retrovirus, das das virale Rückgrat-Protein darstellt, ist in einer bevorzugten Ausführungsform der Erfindung das transmembrane Hüllprotein p15E eines Gammaretrovirus, vorzugsweise FeLV p15E.

In einer weiteren bevorzugten Ausführungsform der Erfindung ist die zweite Aminosäuresequenz ausgewählt aus der Gruppe der Aminosäuresequenzen der SEQ ID NOs: 6, 13 bis 23, 25, 27, 29, 31, 33, 35, 37, 39, 41, 43, 45, 47, 49, 51, 53, 55, 57, 59, 61, 63, 65, 66 bis 71, 83 bis 86 und 88, oder sie codierender DNA, oder Varianten, Mutanten, Teile der Aminosäuresequenzen oder zu 80 % homologe Sequenzen, die die gleichen Eigenschaften besitzen.

In noch einer weiteren bevorzugten Ausführungsform ist die erste Aminosäuresequenz das Hüllprotein zwischen der Fusionsdomäne und der Cystein-Cystein-Schleife. In einer besonders bevorzugten Ausführungsform umfasst die erste Aminosäuresequenz eine Aminosäuresequenz der SEQ ID NOs: 1 bis 5, 7 bis 12, 24, 26, 28, 30, 32, 34, 36, 38, 40, 42, 44, 46, 48, 50, 52, 54, 56, 58, 60, 62, 64, 72 bis 82, 87 und 89, oder sie codierende DNA, oder Varianten, Mutanten, Teile der Aminosäuresequenzen oder zu 80 % homologe Sequenzen, die die gleichen Eigenschaften besitzen. In einer ganz besonders bevorzugten Ausführungsform ist die erste Aminosäuresequenz eine Aminosäuresequenz der SEQ ID NOs: 1 bis 5, 7 bis 12, 24, 26, 28, 30, 32, 34, 36, 38, 40, 42, 44, 46, 48, 50, 52, 54, 56, 58, 60, 62, 64, 72 bis 82, 87 und 89, oder sie codierende DNA, oder Varianten, Mutanten, Teile der Aminosäuresequenzen oder zu 80 % homologe Sequenzen, die die gleichen Eigenschaften besitzen.

Diese Sequenzen stellen eine Teilmenge aus bekannten größeren Aminosäuresequenzbereichen innerhalb retroviraler transmembraner Hüllproteine dar. Die erfindungsgemäße Auswahl der ersten Aminosäuresequenz führt zu dem überraschenden Ergebnis der Induktion neutralisierender Antikörper, wenn sie zusammen mit einer der o.g. zweiten Aminosäuresequenzen eingesetzt wird, gegen die die Antikörper gerichtet sind. Die Auswahl aus der Gruppe, die die Sequenzen aus dem zweiten Bereich beinhaltet, bestimmt dabei das Ziel der neutralisierenden Antikörper, d.h. den zu attackierenden Virus. Die gewählte zweite Aminosäuresequenz kann mit jeder Aminosäuresequenz aus der Gruppe, welche die Sequenzen des ersten Bereichs betrifft und die Vorteile der Erfindung zur Geltung bringt, kombiniert werden. Insbesondere kann im Rahmen eines Screening diejenige Sequenz ermittelt werden, die als Bestandteil des immunogenen Konstrukts die besten Eigenschaften im Sinne der Erfindung aufweist, wie z.B. einen hohen Antikörpertiter.

Bevorzugt ist eine erste Aminosäuresequenz der SEQ ID NOs: 58, 78 und 79, und eine zweite Aminosäuresequenz, die aus der Gruppe der Aminosäuresequenzen der SEQ ID NOs: 6, 13 bis 23, 66 bis 71 und 83 bis 85 ausgewählt ist. Besonders bevorzugt sind die erste Aminosäuresequenz der SEQ ID NO: 78 und die zweite Aminosäuresequenz der SEQ ID NO: 66.

Die Aminosäuresequenzen der genannten SEQ ID NOs sind erfindungsgemäß also dem ersten oder dem zweiten Bereich retroviraler transmembraner Hüllproteine zugeordnet. Bevorzugt wird die zweite Aminosäuresequenz aus gp41 oder gp36 von HI-Viren ausgewählt, so dass es sich bei den bevorzugten Peptiden mit den Aminosäuresequenzen der SEQ ID NOs: 6, 13 bis 23, 66 bis 71 und 83 bis 85, oder sie codierender DNA um solche handelt, die den Aminosäuren 650 bis 683 (C-terminale Sequenz) des HIV-1 Referenz-Genoms (NCBI Datenbank: K03455, HIV HXB2) entsprechen, oder Fragmenten bzw. Untereinheiten hiervon, die funktionsanalog zu den oben genannten Domänen sind. Die die SEQ ID NOs: 67 bis 71 enthaltenen Aminosäuresequenzen sind hierin erstmalig als Epitop-Region bzw. Epitop charakterisiert.

Dem Fachmann ist selbstverständlich bekannt, dass aufgrund der Variabilität bei unterschiedlichen Subtypen von HIV-1 Sequenzvariationen innerhalb dieser Sequenzen vorliegen. Derartige Sequenzvariationen sind von der Erfindung mit erfasst. Dazu werden simultan rekombinante Proteine, die alle Formen der bekannten E2-Regionen umfassen, eingesetzt. Dies trifft auch für Teilsequenzen der Konsensussequenz 650 bis 683 zu, einschließlich chemischer Modifikationen einzelner Aminosäuren, der Verknüpfung der Sequenzen mittels Linker und Sequenzen mit zusätzlichen angefügten Aminosäuren zum Zwecke der Multimerisierung der Sequenzen. Gleiches gilt für die bevorzugten ersten Aminosäuresequenzen der SEQ ID NOs: 58, 78 und 79, die aus FeLV p15E stammen.

Der Fachmann ist mit verschiedene Methoden vertraut, äquivalente Peptide zu generieren, die zu den Peptiden der erfindungsgemäßen Lehre funktionsanalog sind. Die Erfindung schließt deshalb auch Varianten, Mutanten, Teile der Aminosäuresequenzen oder zu mindestens 80 % homologe Sequenzen, die funktionsanalog sind, ein. Mutanten können beispielsweise durch Substitution, Deletion, Insertion, Translokation, Inversion und/oder Addition von zumindest einem Aminosäurerest erhalten werden. Es ist z.B. bekannt, dass einzelne Aminosäuren analoge physikochemische Eigenschaften haben, die mit Vorteil dazu führen, dass diese Aminosäuren untereinander substituiert werden können. Hierzu gehören beispielsweise die Aminosäuren Glycin, Alanin, Valin, Leucin und Isoleucin (a); die Aminosäuren Serin und Threonin (b); die Aminosäuren Asparagin und Glutamin (c), die Aminosäuren Asparaginsäure und Glutaminsäure (d); die Aminosäuren Lysin und Arginin (e) sowie die aromatischen Aminosäuren Phenylalanin, Tyrosin und Tryptophan (f). Aminosäuren innerhalb ein und derselben Gruppe (a bis f) können untereinander ausgetauscht werden. Varianten der erfindungsgemäßen Peptide können durch Modifikationen, wie z.B. Alkylierung, Arylierung oder Acetylierung von zumindest einem Aminosäurerest, Einbau von Enantiomeren und/oder durch Fusion der Peptide mit einer oder mehreren Aminosäuren oder einem Peptid entstehen. Teile der Aminosäuresequenzen beschränken sich auf jene Abschnitte, die für die Expression einer bestimmten Funktion ausreichend sind. Sämtliche Veränderungen der Sequenz sind zwangsläufig durch das Erfordernis des Funktionserhalts beschränkt. Vorzugsweise weist eine Aminosäuresequenz, die zu einer der Aminosäuresequenzen der SEQ ID NOs: 1 bis 89 funktionsanalog ist, eine Homologie von mindestens 60 % auf, besonders bevorzugt von mindestens 70 %, ganz besonders bevorzugt von mindestens 80 %. Für die aufgezeigten Veränderungen derartiger Aminosäuresequenzen sei auf die entsprechenden Standardwerke der Biochemie und der Molekularbiologie verwiesen. Im Stand der Technik sind darüber hinaus verschiedene Möglichkeiten der Generierung nicht homologer, aber funktionsanaloger Peptide offenbart. Sämtliche Peptide, Peptidfragmente oder Strukturen, die Peptide umfassen, die von den erfindungsgemäßen Peptiden ausgehend mit solchen Verfahren entwickelt werden, sind von der erfindungsgemäßen Lehre mit erfasst und damit Peptide im Sinne der Erfindung, sofern sie die erfindungsgemäße Aufgabe lösen, insbesondere neutralisierende Antikörper zu generieren. Eine Möglichkeit des Generierens von funktionsanalogen Peptiden ist beispielsweise in Schneider et al. (1998) PNAS USA 95(21), 12179-12184, in WO 99/6293 und WO 02/38592 beschrieben. Diese Lehren sind in den Offenbarungsgehalt der Erfindung mit aufgenommen.

Die Aminosäuresequenzen und die sie codierende DNA sind untereinander verbunden oder assoziiert. Es hat sich herausgestellt, dass eine antiparallele Anordnung der Aminosäuresequenzen die Immunogenität des Konstrukts signifikant erhöht. Für die Präsentation der ausgewählten zweiten Aminosäuresequenz ist insbesondere eine räumliche Nähe zu der ersten Aminosäuresequenz notwendig, so dass im erhaltenen strukturellen Kontext wirkungsvoll neutralisierende Antikörper induziert werden. Die Interaktion beider Bereiche wird vorteilhaft durch eine kovalente Kopplung erzielt, die sich durch eine feste Verknüpfung von zwei Komponenten auszeichnet.

Die Assoziation zu einer Struktur, die neutralisierende Antikörper induziert, wird insbesondere durch die natürliche Faltung des Rückgrats eines retroviralen transmembranen Hüllprotein gefördert. Das als Linker zu verwendende Rückgrat umfasst einen Bereich eines retroviralen transmembranen Hüllproteins, der zwischen der N-terminalen und der C-terminalen Helix-Region lokalisiert ist, oder einen Teil davon, wobei das Hüllprotein beliebig gewählt werden kann. Beispielsweise handelt es sich um das Hüllprotein des ersten Retrovirus, das die erste Aminosäuresequenz umfasst, oder das Hüllprotein des zweiten Retrovirus, aus dem die zweite Aminosäuresequenz ausgewählt ist, oder um ein anderes Hüllprotein des ersten oder zweiten Retrovirus. Selbstverständlich kann es sich beim Linker auch um ein Hüllprotein eines dritten Virus handeln, das eine Sequenz-, Struktur- und/oder Funktionshomologie zu den Hüllproteinen des ersten bzw. zweiten Retrovirus im Sinne der Erfindung aufweist oder nicht. Des weiteren kann der Rückgrat-basierende Peptid-Linker auch aus mehreren Hüllproteinen oder Teilen mehrerer Hüllproteine zusammengesetzt sein. Die Auswahl eines geeigneten Rückgrats als Linker wird physikochemische Kriterien, wie z.B. die Löslichkeit oder Größe des entstehenden immunogenen Konstrukts, und/oder hinsichtlich der Immunantwort eine hohe Induktion neutralisierender Antikörper im Sinne der Erfindung bei gleichzeitig minimalen Nebenwirkungen berücksichtigen.

Bevorzugt sind die erste und die zweite Aminosäuresequenz durch ein Hüllprotein-Rückgrat verbunden, der einen Bereich, der zwischen der N-terminalen und der C-terminalen Helix-Region des Hüllproteins des ersten Retrovirus lokalisiert ist, oder einen Teil davon umfasst. Das genannte Hüllprotein umfasst also die erste Aminosäuresequenz und den Peptid-Linker inhärent. Der Peptid-Linker kann eine Cystein-Cystein-Schleife umfassen. Das immunogene Konstrukt ist ein rekombinantes Protein, das aus dem transmembranen Hüllprotein des ersten Retrovirus oder einem Teil davon, und der erfindungsgemäß aus einem Hüllproteins eines zweiten Retrovirus ausgewählten zweiten Aminosäuresequenz besteht, ein sogenanntes Hybridprotein. Die ausgewählte zweite Aminosäuresequenz kann durch Rekombinationstechniken auf DNA-Ebene oder durch andere dem Fachmann bekannte Methoden in das Hüllprotein des ersten Retrovirus eingebracht werden, und das Hybridprotein wird anschließend exprimiert.

Es hat sich gezeigt, dass die zusätzliche Deletion der immunsuppressiven Domäne isu (Cianciolo et al., Science 230, 453-455 (1985)) und der Peptide J und K (Nick et al., J. Gen. Virol. 71, 77-83 (1990)) im Hüllprotein des ersten Retrovirus möglich ist. Gleichzeitig mit der in der N-terminalen Helix-Region von Retroviren konservierten isu-Domäne ist eine weitere Domäne gleicher Länge aus der C-terminalen Helix-Region zu entfernen. Diese Veränderungen des Rückgrats im ersten Hüllprotein führen auch zum Verlust des Peptids J in der NHR und des Peptids K in der CHR, die nachweislich "enhancing antibodies" induzierten, und einer vorteilhaft geringen Größe sowohl des Rückgrats als auch des immunogenen Gesamtkonstrukts.

Die Fixierung der ersten und zweiten Aminosäuresequenz ist auch durch einen Peptid-Linker möglich, vorzugsweise mit einer Länge von 1 bis 100 Aminosäuren, besonders bevorzugt von 10 bis 50 Aminosäuren. Peptidbindungen sind stabil und starr, so dass sowohl Festigkeit als auch eine geringe strukturelle Flexibilität gegeben sind. Darüber hinaus findet eine definierte Faltung statt, die die gewünschte strukturelle Assoziation der Aminosäuresequenzen und infolge Energieminimierung eine hohe Stabilität der Struktur gewährleistet. Peptide des immunogenen Konstrukt können während der Synthese unkompliziert mit einem Peptid-Linker verknüpft werden. Hierbei werden die Sequenzen nach der theoretischen Auswahl de-novo generiert. Beispielsweise kann das gesamte immunogene Konstrukt als rekombinantes Protein exprimiert oder chemisch synthetisiert werden. Die Erfindung beinhaltet auch rekombinante Proteine, die aus zwei N-terminalen und einer C-terminalen Sequenz bestehen oder Hexamere, bestehend aus drei N-terminalen Sequenzen und drei C-terminalen Sequenzen, oder Multimere der zuvor aufgeführten rekombinanten Strukturen, wobei zwischen den N- und den C-terminalen Sequenzen je eine Peptidbrücke von 1 bis 100 Aminosäuren vorhanden sein kann. Die Peptide können zum Zwecke der Aufreinigung, Solubilisierung bzw. Konformationsveränderung mit spezifischen Fusionsanteilen entweder am N- oder am C-Terminus versehen sein, wie zum Beispiel CBP (Calmodulin-Bindungsprotein) oder His-Tag. Ähnliche Konstrukte können auch von DNA, die zum Immunisieren verwendet wird, codiert werden.

Synthetische Peptide können durch chemische Crosslinker oder heterobifunktionale Crosslinker multimerisiert werden oder an ein Trägermolekül, wie z.B. BSA, Dextran, KLH oder andere gekoppelt werden. Geeignete Crosslinker und Trägermoleküle sowie Verfahren zu deren Kopplung mit den ausgewählten Peptiden zum erfindungsgemäßen immunogenen Konstrukt sind dem Fachmann bekannt. Bei den Trägern im Sinne der Erfindung kann es sich um Proteine handeln, die aufgrund ihres immunogenen Verhaltens die Antikörperantwort stimulieren, aber auch um dem Fachmann bekannte pharmazeutische Hilfsstoffe wie z.B. QS21, GPI-0100 oder andere Saponine, Wasser-Öl-Emulsionen, wie z.B. Montanide, Polylysin oder Polyagenin-Verbindungen, Phosphat-gepufferte Kochsalzlösung, Wasser, verschiedene Arten von Detergentien, sterile Lösungen und dergleichen mehr.

Die Erfindung betrifft auch pharmazeutische Mittel, die mindestens eines der erfindungsgemäßen immunogenen Konstrukte umfassen, vorzugsweise zusammen mit pharmazeutisch verträglichen Hilfsstoffen und/oder Trägern. Ein pharmazeutisches Mittel im Sinne der Erfindung ist jedes Mittel im Bereich der Medizin, welches in der Prophylaxe, Diagnose, Therapie, Verlaufskontrolle oder Nachbehandlung von Patienten eingesetzt werden kann, die mit umhüllten Viren so in Kontakt gekommen sind, dass zumindest zeitweise eine pathogene Modifikation des Gesamtzustandes bzw. des Zustandes einzelner Teile des Patientenorganismus auftreten konnte. So ist es beispielsweise möglich, dass das pharmazeutische Mittel im Sinne der Erfindung eine Vakzine oder ein Immuntherapeutikum ist. Das pharmazeutische Mittel kann neben dem immunogenen Konstrukt beispielsweise ein akzeptables Salz oder Komponenten davon umfassen. Hierbei kann es sich beispielsweise um Salze anorganischer Säuren, wie z.B. der Phosphorsäure, oder um Salze organischer Säuren handeln. Die jeweilige Dosis bzw. der Dosisbereich für die Gabe des erfindungsgemäßen pharmazeutischen Mittels ist groß genug, um den gewünschten prophylaktischen oder therapeutischen Effekt der Bildung von neutralisierenden Antikörpern zu erreichen. Hierbei sollte die Dosis nicht so gewählt werden, dass unerwünschte Nebeneffekte dominieren. Im Allgemeinen wird die Dosis mit dem Alter, der Konstitution und dem Geschlecht des Patienten variieren sowie die Schwere der Erkrankung berücksichtigen. Die individuelle Dosis kann sowohl in Bezug auf die primäre Erkrankung als auch in Bezug auf das Eintreten zusätzlicher Komplikationen eingestellt werden. Die exakte Dosis ist durch einen Fachmann mit bekannten Mitteln und Methoden feststellbar. Zur Unterstützung der Immunantwort kann das pharmazeutische Mittel in einer bevorzugten Ausführungsform der Erfindung auch weitere immunogene Komponenten umfassen.

Erfindungsgemäß ist das vorliegende pharmazeutische Mittel zur prophylaktischen oder therapeutischen Behandlung von retroviralen Erkrankungen, vorzugsweise von retroviralen Immunerkrankungen, besonders bevorzugt von HIV geeignet. Die vorherige Lehre der Erfindung und deren Ausführungsformen sind gültig und ohne Einschränkungen auf pharmazeutische Mittel anwendbar, sofern es sinnvoll erscheint.

Das Einbringen des pharmazeutischen Mittels in einen Organismus kann erfindungsgemäß auf jede Art und Weise geschehen, die es ermöglicht, dass das Immunsystem mit dem enthaltenen immunogenen Konstrukt in Kontakt gebracht und eine Antikörperantwort induziert wird. Das pharmazeutische Mittel der vorliegenden Erfindung kann oral, transmucosal, rektal, enteral und/oder parenteral angewendet werden. Bevorzugt ist aber eine direkte Injektion in den Körper. Die gewählte Art der Verabreichung richtet sich nach der Indikation, der zu verabreichenden Dosis, Individuum-spezifischen Parametern etc. Insbesondere ermöglichen die verschiedenen Arten der Verabreichung eine ortspezifische Therapie, die Nebenwirkungen minimiert und die Wirkstoffdosis verringert. Bevorzugt sind die Schluckimpfung oder die Impfung durch Injektion. Bevorzugte Injektionen sind die intradermale, subkutane, intramuskuläre oder intravenöse Injektion. Die Impfung erfolgt vorteilhafterweise so, dass es nach der Applikation im Organismus zur Entwicklung eines aktiven Impfschutzes kommt. Selbstverständlich ist es auch möglich, dass die Impfung unmittelbar vor oder zeitnah nach der Infektion erfolgt oder als Therapie mehrfach appliziert wird. Dem Fachmann ist bekannt, dass die Induktion neutralisierender Antikörper zu fast jedem Zeitpunkt auch nach der Infektion von Vorteil sein kann, so dass eine Impfung im Sinne der Erfindung auch eine Applikation des erfindungsgemäßen pharmazeutischen Mittels nach der Infektion mit dem jeweiligen Virus sein kann.

Selbstverständlich ist es auch möglich, das immunogene Konstrukt als Aerosol bereitzustellen, welches von dem Organismus, bevorzugt einem humanen Patienten, inhaliert wird. Dem Fachmann sind ebenfalls Verfahren zum Aufbringen von Antigenen auf Schleimhäute bekannt. Sofern das pharmazeutische Mittel für die Immunisierung ein immunogenes DNA-Konstrukt beinhaltet, kann die Applikation z.B. mit Hilfe sogenannter Impfpistolen, die die DNA mittels Goldkugeln in die Haut einbringen, oder mittels Spritzen, die die DNA unter die Haut oder in den Muskel einbringen, geschehen.

Um die protektive oder therapeutische Wirkung der erfindungsgemäßen immunogenen Konstrukte, d.h. die Induktion von neutralisierenden Antikörpern zu erhöhen, können dem immunogenen Konstrukt bzw. allen pharmazeutischen Mitteln, die aus diesem hergestellt werden, insbesondere dem Impfstoff (Vakzine), pharmazeutisch verträgliche Hilfsstoffe, wie z.B. Adjuvantien, zugesetzt werden. Im Sinne der Erfindung ist jede Substanz, die bei gleichzeitiger, zeitnaher oder zeitversetzter Verabreichung mit den erfindungsgemäßen Peptiden oder Proteinen eine spezifische Immunantwort gegen diese ermöglicht, verstärkt oder modifiziert, ein Adjuvanz. Bekannte Adjuvantien für Humanvakzine sind beispielsweise Aluminiumverbindungen, wie z.B. Aluminiumhydroxid oder Aluminiumphosphat, Saponine, wie z.B. QS 21, Muramyldipeptid oder Muramyltripeptid, Proteine, wie z.B. Gammainterferon oder TNF, MF 59, Phosphatdibylcholin, Squalen oder Polyole. Ebenfalls kann die Co-Applikation von Ei-Albumin in komplettem Freund'schen Adjuvanz eine gesteigerte zellvermittelte Immunität hervorrufen und somit die Wirkung neutralisierender Antikörper unterstützen. Dasselbe trifft für die für die Immunisierung verwendeten DNA-Konstrukte zu. Hier kann DNA, die selbst eine immunstimulatorische Eigenschaft hat, oder die ein Protein mit Adjuvanz-Effekt, wie z.B. ein Cytokin, codiert, parallel oder in einem Konstrukt appliziert werden.

Das pharmazeutische Mittel kann als Tablette, Kapsel, Pulver, Lösung, Dispersion oder Suspension vorliegen. Die Darreichungsformen des pharmazeutischen Mittels werden mit den üblichen festen oder flüssigen Trägerstoffen und/oder Verdünnungsmitteln und den üblicherweise allgemein eingesetzten Hilfsstoffen entsprechend der gewünschten Applikationsart in einer geeigneten Dosierung und in an sich bekannter Weise hergestellt. Als orale Darreichungsform werden vorzugsweise Tabletten, Filmtabletten, Dragees, Kapseln, Pillen, Pulver, Lösungen oder Suspensionen - auch als Depotform - zubereitet. Arzneiformen als Tabletten können beispielsweise durch Mischen des Wirkstoffes mit bekannten Hilfsstoffen, wie Dextrose, Zucker, Sorbit, Mannit, Polyvinylpyrrolidon, Sprengmitteln, wie Maisstärke oder Alginsäure, Bindemitteln, wie Stärke oder Gelatine, Gleitmitteln, wie Magnesiumstearat oder Talk, und/oder Mitteln, die einen Depoteffekt erzielen können, wie Carboxypolymethylen, Carboxymethylcellulose, Celluloseacetatphthalat oder Polyvinylacetat, erhalten werden. Die Tabletten können auch aus mehreren Schichten bestehen. Ebenso lassen sich Dragees durch Überziehen von analog zu den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker bereiten. Die Drageehülle kann dabei auch aus mehreren Schichten bestehen, wobei beispielsweise die oben genannten Hilfsstoffe verwendet werden. Die Kapseln können durch Mischen des Wirkstoffes mit Trägern, wie Milchzucker oder Sorbit, hergestellt werden, die dann in Kapseln eingebracht werden. Die Lösungen, Dispersionen oder Suspensionen des pharmazeutischen Mittels können zur Verbesserung des Geschmacks mit Stoffen, wie z.B. Saccharin, Cyclamat oder Zuckerarten, und/oder mit Aromastoffen, wie z.B. Vanillin oder Orangeextrakt, versetzt werden. Weiterhin können sie mit Suspendierhilfsstoffen, wie z.B. Natriumcarboxymethylcellulose, oder Konservierungsmitteln, wie z.B. p-Hydroxybenzoesäure, Phenol, Benzylalkohol, m-Kresol, Methylparaben, Propylparaben, Benzalkoniumchlorid oder Benzethoniumchlorid, vermischt werden. Des weiteren sind parenterale Arzneiformen, wie Suppositorien, in Betracht zu ziehen. Zur parenteralen Verabreichung kann das immunogene Konstrukt der Erfindung in einem physiologisch verträglichen Verdünnungsmittel gelöst oder suspendiert werden, wie z.B. Neutralfetten oder Polyethylenglykolen oder dessen Derivaten. Als bevorzugte Lösungsmittel werden häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnussöl, Baumwollsamenöl, Rizinusöl und Sesamöl. Zur topischen Applikation des pharmazeutischen Mittels wird dieses mit mindestens einem pharmazeutisch annehmbaren Trägerstoff, wie z.B. mikrokristalliner Cellulose, und ggf. weiteren Hilfsstoffen, wie z.B. Feuchtigkeitsspendern, zu auf die Haut auftragbaren festen Formulierungen, wie z.B. Cremes, Gelen, Salben oder Emulsionen bzw. zu auf die Haut auftragbaren flüssigen Formulierungen, wie z.B. Lösungen, Suspensionen, Lotionen, Seren oder Ölen, in üblicher Weise formuliert. Als Trägersysteme für das pharmazeutische Mittel können auch Liposomen oder Nanopartikel dienen, die einen optimalen Transport in die Haut gewährleisten. Grundsätzlich können pharmazeutisch annehmbare und dem Fachkundigen bekannte Exzipienten einen Teil der erfindungsgemäßen Vakzinen oder Formulierungen bilden. Diese Exzipienten umfassen beispielsweise auch Salze, verschiedene Füller, zusätzliche Pufferagenzien, Chelatbildner, Antioxidantien, Co-Lösungsmittel und dergleichen.

Gegenstand der vorliegenden Erfindung sind auch neutralisierende Antikörper, die durch Immunisierung mit dem erfindungsgemäßen immunogenen Konstrukt hergestellt sind. Bei der Herstellung der neutralisierenden Antikörper wird mindestens ein erfindungsgemäßes immunogenes Konstrukt so mit einem Organismus in Kontakt gebracht, dass dieser in der Lage ist, gegen dieses Konstrukt Antikörper herzustellen, die durch den Fachmann mit den bekannten Methoden gewonnen werden können.

Ein weiterer Gegenstand der Erfindung betrifft die Verwendung dieser neutralisierenden Antikörper zur Diagnose, Prophylaxe, Therapie und/oder Verlaufskontrolle von retroviralen Erkrankungen, vorzugsweise HIV. Die neutralisierende Wirkung der Antikörper auf umhüllte Retroviren in Hinsicht auf ihr prophylaktisches Potential zeigt sich z.B. als Inhibition der Virusinfektion, als Inhibition der Syncytiumbildung, als Inhibition der Fusion zwischen Virus und Target-Membran oder als Verminderung oder Stabilisierung der Vermehrungsrate der Retroviren in einem Organismus. Die neutralisierende Wirkung in Hinsicht auf ihre therapeutische Wirkung kann beispielsweise darin bestehen, dass durch die Induktion oder Applikation der Antikörper als erwünschter Nebeneffekt bestimmte anti-virale Medikamente besser wirken oder durch die Verminderung der Dosis die Anzahl der Nebenwirkungen dieser Medikamente reduziert wird. Die Wirkung der Antikörper im Sinne der Erfindung ist nicht auf eine Eliminierung der Retroviren beschränkt, sondern umfasst das gesamte Spektrum vorteilhafter Wirkungen in einer Therapie bzw. Prophylaxe. Selbstverständlich kann auch das erfindungsgemäße immunogene Konstrukt zur Diagnose, Prophylaxe, Therapie und/oder Verlaufskontrolle von retroviralen Erkrankungen, vorzugsweise HIV verwendet werden.

Noch ein weiterer Gegenstand der Erfindung ist ein immunologisches Verfahren für den Nachweis von HIV-1-, FeLV-, BIV-, CEAV-, EIAV1-, FIV-, OMVV-, SIVmac-, SIVcpz-, VILV-, HIV-2-, RSV-, ALV-, JSRV-, SRV-, GALV-, MLV-, BLV-, HTLV-1-, HTLV-2- und/oder KoRV-Antikörpern in einer biologischen Probe, umfassend
a) Beschichten einer festen Phase mit dem erfindungsgemäßen immunogenen Konstrukt,
b) Inkubieren der festen Phase mit der biologischen Probe,
c) Inkubieren der festen Phase mit einem anti-humanen Antikörper, der die Klassen IgA, IgM, IgG nachweisen kann und der mit einer nachweisbaren Kennzeichnung markiert ist, und
d) Nachweis der Kennzeichnung zur Bestimmung der Anwesenheit bindender Antikörper gegen die genannten Retroviren in der Probe.

Die feste Phase können beispielsweise Platten, Streifen, Membrane, Filme, Gele oder Perlen sein. Geeignete Trägermaterialien sind anorganische und organische Polymere. Hierzu zählen Kunststoffe, vorzugsweise auf Basis von Polystyrol. Des weiteren sind Biopolymere, vorzugsweise Cellulose, Dextran, Agar, Agarose und Sephadex, die funktionalisiert sein können, insbesondere als Nitrocellulose und Cyanbromid-Sephadex, Polymere im erfindungsgemäßen Verfahren. Ebenfalls finden Gläser, vorzugsweise auf Basis von Siliziumdioxid, die ggf. porös sind und die silanisierte Oberflächen aufweisen können, Verwendung im erfindungsgemäßen Verfahren. Diese Beispiele für Polymere sind nicht limitierend und andere Moleküle denkbar. Bevorzugte Kombinationen von geometrischer Form und Material sind Platten aus Kunststoff, wie sie z.B. handelsübliche 12-, 24, 48-, 96-Well-Platten oder andere Plattenformate darstellen, Streifen aus Papier, das auf Cellulose als Grundstoff basiert, Gele aus Biopolymeren, insbesondere Agarose, und Perlen aus Dextran und Sephadex, die auch Hohlräume definierter Porengrößen ausbilden.

Das erfindungsgemäße immunogene Konstrukt wird vorzugsweise auf der festen Phase immobilisiert. Geeignete Methoden der Immobilisierung sind dem Fachmann bekannt. Das immunologische Verfahren kann dabei als Microarray-System konzipiert sein, wobei die Peptide des immunogenen Konstrukts direkt auf die Chip-Oberfläche synthetisiert oder gespottet werden.

Anschließend wird die feste Phase mit einer Probe inkubiert. Eine "Probe" im Sinne der Erfindung ist die Bezeichnung für ein durch Probenentnahme entnommenes biologisches oder chemisches Gut oder eines Teiles bzw. einer kleinen Menge eines solchen, dessen Beschaffenheit chemisch, biologisch, klinisch oder ähnlich geprüft werden soll, insbesondere auf das Vorhandensein von neutralisierenden Antikörpern. Eine Probe können insbesondere alle biologischen Materialien sein, die von einem Individuum oder Individuen isoliert worden sind, beispielsweise biologische Gewebe und Flüssigkeiten, zu denen u.a. Blut, Haut, Plasma, Serum, Lymphe, Urin, Gehirnflüssigkeit, Tränen, Abstriche, Gewebeproben, Organe und Tumore zählen. Vorliegend sind in Proben auch Bestandteile von Zellkulturen eingeschlossen. Die Probenentnahme erfolgt insbesondere so, dass die entnommene Teilmenge einem Durchschnitt der gesamten Menge entspricht. Die durch Untersuchung der Probe ermittelten Merkmale dienen der Beurteilung der durch die Probe erfassten Menge, die wiederum Rückschlüsse auf die Gesamtmenge, beispielsweise das Blut bzw. die Lymphe in einem Organismus, zulässt. Für die Untersuchung können die Proben durch Mischen, Zugabe von Enzymen oder Markern oder anders vorbehandelt werden.

In der Probe vorhandene anti-virale Antikörper und das immunogene Konstrukt (Antigen) bilden einen Komplex. Das sogenannte Präzipitat oder Agglutinat ist meist nicht direkt detektierbar und wird vorzugsweise in einer Indikatorreaktion nach einer direkten oder indirekten Kopplung eines Komplexpartners mit einer gut nachweisbaren Markierungssubstanz sichtbar gemacht. Zunächst kann der vorliegende Antigen-Antikörper-Komplex mit einem anti-humanen Antikörper, der gegen die humanen antiviralen Antikörper (Liganden) aus der Probe gerichtet ist, präzipitiert werden. Bei dem anti-humanen Antikörper kann es sich um einen polyklonalen oder einen monoklonalen Antikörper handeln, der mit gängigen Techniken, wie z.B. der Hybridoma-Technologie, erhalten wird. Vorzugsweise ist der anti-humane Antikörper gegen die Klassen IgA, IgM und/oder IgG gerichtet. Die Komplexe aus zwei Antikörpern und einem Antigen heißen Sandwichkomplexe, die nachfolgend mit einer Indikatorreaktion nachgewiesen werden. Ist beispielsweise der anti-humane Antikörper an ein Enzym gebunden, spricht man vom ELISA (enzyme-linked immunsorbend assay). Die Detektion erfolgt durch eine nachgeschaltete enzymatische Umsetzung eines Substrates zu einem vorzugsweise farbigen Produkt, dass z.B. durch visuelle Farbgebung, Biolumineszenz, Fluoreszenz oder die Messung elektrischer Signale (Enzymelektrode) erfasst wird. Zur Markierung verwendete Enzyme sind dem Fachmann bekannt und umfassen vorzugsweise Peroxidase, CAT, GFP, GST, Luciferase, β-Galactosidase oder Alkalische Phosphatase. Aufgrund der Enzymstabilität und einer Vielzahl an möglichen Substraten ist die Meerettich-Peroxidase bevorzugt. Die vorherige kovalente Verknüpfung (Konjugation) eines Antikörpers mit einem entsprechenden Enzym kann auf verschiedene Arten erfolgen. Eine Methode ist die Kopplung mittels Glutaraldehyd, bei der sowohl das Enzym als auch der Antikörper über freie Aminogruppen an das Glutaraldehyd gebunden werden. Wenn das Enzym ein Glycoprotein ist, wie z.B. die Peroxidase, kann es über Zuckerreste an den Antikörper gekoppelt werden. Diese Methode kommt ohne einen zusätzlichen Linker aus. Die Kopplung des Enzyms kann auch über einen heterobifunktionalen Linker, wie z.B. MHS (Maleimido-Hexansäure-N-hydroxysuccinimid) erfolgen. Dabei werden die NH₂-Gruppen des Antikörpers mit freien SH-Gruppen eines Enzyms, wie z.B. der β-Galactosidase, schonend verknüpft. Alternativ kann der Nachweis auch radioaktiv in einem RIA (radioactive immunoassay) mit radioaktiven Isotopen, vorzugsweise mit ¹²⁵I, oder durch Fluoreszenz in einem FIA (Fluoroimmunoassay) mit Fluorophoren, vorzugsweise mit Fluorescein oder FITC, erfolgen. Alle genannten Verfahren schließen intensive Waschschritte ein, um ungebundene und/oder unspezifisch gebundene Antikörper abzutrennen. Die Durchführung sämtlicher Nachweisverfahren ist dem Fachmann bekannt.

In einem nachgeschalteten Infektionsassay kann die Subpopulation der neutralisierenden Antikörper ermittelt werden, indem die Hemmung der Infektion von uninfizierten Zellen durch das jeweilige Retrovirus aus der obigen Liste durch die neutralisierenden Antikörper nachgewiesen wird. Insbesondere durch Kompetitionsanalysen mit dem immunogenen Konstrukt kann aus der Menge der neutralisierende Antikörper jener Anteil, der spezifisch gegen die Epitope auf dem immunogenen Konstrukt gerichtet ist, quantitativ bestimmt werden. Da eine Korrelation zwischen der Menge der Antikörper, die das immunogene Konstrukt bindet, und der Menge der neutralisierenden Antikörper gefunden wurde, kann ein neuer Schnelltest durchgeführt werden, in dem erstmals in kurzer Zeit viele Seren auf neutralisierende Antikörper getestet werden können. Dieser Test erlaubt Aussagen über den Erfolg der Immunisierung im Fall einer prophylaktischen Applikation wie auch über die Progression der Infektion. Auch ein Überwachen des Therapieerfolges im Fall der therapeutischen Applikation ist möglich. Dem gemäß kann auch ein neuartiges Schnellverfahren zum Nachweis neutralisierender Antikörper auf der Basis eines ELISA mit dem immunogenen Konstrukt bereitgestellt werden.

Die Erfindung betrifft auch ein immunologisches Verfahren für den Nachweis von retroviralen Antigenen zur Bestimmung der Retrovirusbelastung in den oben aufgelisteten biologischen Proben, umfassend
a) Beschichten einer festen Phase mit mindestens einem neutralisierenden Antikörper, der nach der Immunisierung mit dem erfindungsgemäßen Konstrukt induziert und gewonnen wurde,
b) Inkubieren der festen Phase mit der biologischen Probe,
c) Inkubieren der festen Phase mit einem vom ersten unterschiedlichen zweiten Antikörper gegen die gesuchten retroviralen Antigene, wobei der zweite Antikörper in einem Organismus nach Immunisierung mit dem immunogenen Konstrukt gewonnen wurde, und
d) Nachweis des gekoppelten zweiten Antikörpers zur Bestimmung der Anwesenheit des Antigens in der Probe.

Der neutralisierende Antikörper wird gemäß der vorherigen Lehre dieser Erfindung induziert und mit dem Fachmann bekannten Methoden aufgereinigt. Die Auswahl der festen Phase und die Beschichtung mit dem neutralisierenden Antikörper können analog zur o.g. Auswahl und der Beschichtung mit dem immunogenen Konstrukt erfolgen. Zur Immobilisierung von Antikörpern sei auf die Standard-Lehrbücher der Biochemie verwiesen. Die beschichtete Phase wird mit einer biologischen Probe inkubiert, von der vermutet wird, dass sie retrovirale Antigene enthält, so dass Präzipitate entstehen können. Der Ligand in Form des retroviralen Antigens wird mit einem vom ersten unterschiedlichen zweiten Antikörper, der gegen ein anderes Epitop des Antigens gerichtet ist, inkubiert. Sowohl der erste als auch der zweite Antikörper können durch Immunisierung in einem Tier oder einem Menschen induziert und gewonnen werden. Während der erste Antikörper neutralisierende Eigenschaften aufweist, sind beim zweiten Antikörper bindende Eigenschaften ausreichend. Der zweite Antikörper ist vorteilhaft mit einer nachweisbaren Kennzeichnung markiert. Die vorherige Lehre der Erfindung und deren Ausführungsformen sind gültig und ohne Einschränkungen auf den Nachweis des gekoppelten zweiten Antikörpers anwendbar. Die Bestimmung des gebundenen Antigens lässt Rückschlüsse auf die Menge an retroviralen Antigenen in der Probe und damit auf den Virustiter zu.

Gegenstand der vorliegenden Erfindung ist auch ein Verfahren zur Induktion einer Antikörperantwort, dadurch gekennzeichnet, dass das erfindungsgemäße immunogene Konstrukt einem Organismus appliziert und die Bildung neutralisierender Antikörper induziert wird. Ein weiterer Gegenstand der Erfindung ist ein Verfahren zur Induktion einer Antikörperantwort, dadurch gekennzeichnet, dass das erfindungsgemäße pharmazeutische Mittel einem Organismus appliziert und die Bildung neutralisierender Antikörper induziert wird. Bevorzugte Organismen im Sinne der Erfindung sind Mensch oder Tier. Bevorzugt umfasst das Verfahren die Formulierung des neutralisierenden Antikörpers mit einem pharmazeutisch akzeptablen Träger. Durch die Offenbarung der erfindungsgemäßen Peptide ist es dem Fachmann möglich, diese als Antigen zur Induktion von neutralisierenden Antikörpern zu verwenden. Dem Fachmann ist hierbei bekannt, dass er die erfindungsgemäßen Peptide, die selbstverständlich auch als das erfindungsgemäße pharmazeutische Mittel verwendet werden können, in verschiedenen Dosierungen einem Organismus, insbesondere einem humanen Patienten, applizieren kann. Die Applikation sollte hierbei so erfolgen, dass eine möglichst große Menge neutralisierender Antikörper generiert wird. Die Konzentration und die Art der Applikation kann vom Fachmann durch Routineversuche eruiert werden. Möglich sind beispielsweise folgende Applikationen: oral in Form von Pulver, Tabletten, Saft, Tropfen, Kapseln und ähnlichem; rektal in Form von Zäpfchen, Lösungen und ähnlichem; parenteral in Form von Injektionen, Infusionen von Lösungen, Inhalationen von Dämpfen, Aerosolen, Stäuben und Pflastern sowie lokal in Form von Salben, Pflastern, Umschlägen, Spülungen und ähnlichem. Das In-Kontakt-Bringen des immunogenen Konstruktes oder des pharmazeutischen Mittels kann prophylaktisch oder therapeutisch erfolgen. Bei einer prophylaktischen Impfung soll die Infektion mit den genannten Retroviren zumindest dergestalt verhindert werden, dass nach Eindringen einzelner Retroviren, beispielsweise in eine Wunde, eine weitere Vermehrung dieser stark vermindert wird oder dass eingedrungene Retroviren nahezu vollständig abgetötet werden. Bei einer therapeutischen Induktion einer Immunantwort liegt bereits eine Infektion des Patienten vor und die Induktion der neutralisierenden Antikörper erfolgt, um die bereits im Körper befindlichen Retroviren abzutöten bzw. in ihrer Vermehrung zu hemmen.

Noch ein weiterer Gegenstand der Erfindung ist ein Verfahren zur passiven Immunisierung eines Organismus, dadurch gekennzeichnet, dass die Antikörper, die durch das Applizieren des erfindungsgemäßen immunogenen Konstrukts mit einem Organismus induziert wurden, gewonnen und einem weiteren Organismus appliziert werden. Unter einem "weiteren Organismus" im Sinne der Erfindung sind sowohl artgleiche als auch artfremde Organismen zu verstehen, jedoch nicht derselbe Organismus, der die genannten Antikörper induziert hat. Es können auch monoklonale Antikörper gewonnen werden, die u.a. nach entsprechender Humanisierung eingesetzt werden. Dabei können ebenfalls Antikörper-produzierende Zellen aus geimpften oder infizierten Individuen gewonnen werden, die neutralisierende Antikörper produzieren, die gegen das erfindungsgemäße immunogene Konstrukt gerichtet sind, und in Form monoklonaler Antikörper bei der passiven Immunisierung appliziert werden. Bei der passiven Immunisierung erfolgt im Körper des Patienten im Wesentlichen keine eigene Immunreaktion auf bestimmte Viren, sondern die Antikörper werden beispielsweise in Form von Heilseren in den Patienten eingebracht. Passive Immunisierung hat im Gegensatz zur aktiven Immunisierung die Aufgabe, eine bereits erfolgte Infektion möglichst schnell zu heilen oder aber sofort gegen eine Infektion mit Viren zu schützen. Dem Fachmann sind beispielsweise aus der passiven Immunisierung gegen Hepatitis A, Hepatitis B oder der FSME verschiedene Impfschemata zur passiven Immunisierung bekannt. Derartige Impfschemata können durch Routineversuche auf spezifische Retroviren, wie z.B. HIV, das Feline Leukämievirus und andere adaptiert werden. Die Antikörper, die zur passiven Immunisierung verwendet werden, sind bevorzugt monoklonale Antikörper. Sie werden insbesondere als Bestandteil einer Kombinationstherapie verwendet.

Anschließend soll die Erfindung an einem Ausführungsbeispiel näher erläutert werden.

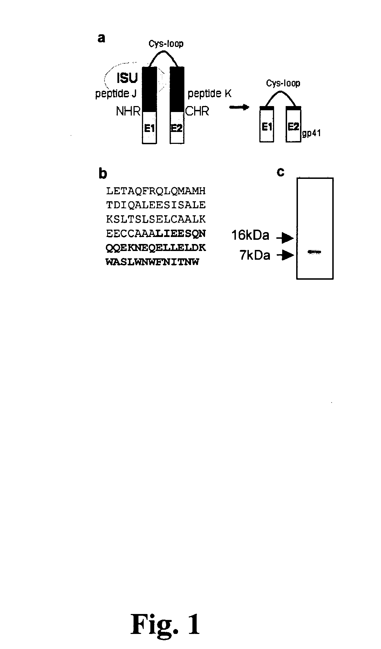

Fig. 1: Charakterisierung des Hybridproteins p15E/gp41. a: Schematische Darstellung der p15E-Ectodomäne von FeLV und des modifizierten Hybridproteins p15E/gp41. Die Lage der isu-Domäne, der Peptide J und K, der Cystein-Cystein-Schleife, der N-terminalen und C-terminalen Helix-Regionen (NHR und CHR) sowie der Epitop-Regionen (E1, E2) sind angegeben. Die weiß abgebildete Epitop-Region kennzeichnet die C-terminale gp41-Sequenz. b: Aminosäuresequenz des p15E/gp41-Hybrids. Die C-terminale gp41-Sequenz ist fett gedruckt. c: Nachweis des Hybridproteins p15E/gp41 im Western Blot mit mAb 2F5 (0,5 mg/ml). 1 µg Suspension des Hybridprotein wurde aufgetragen.

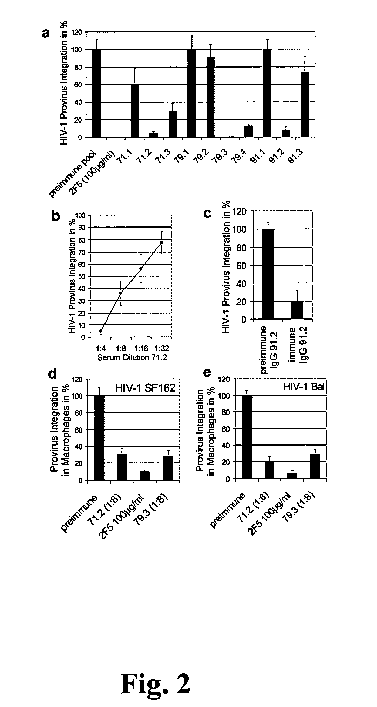

Fig. 2: Neutralisation von HIV-1 IIIB sowie von zwei HIV-1-Primärisolaten mit Ratten-Antiseren, die durch Immunisierung mit dem Hybridprotein p15E/gp41 induziert wurden. Die Virusinfektion wurde mittels quantitativer Realtime-PCR als Integration des Provirus gemessen. mAb 2F5 in einer Konzentration von 100 µg/ml diente als Positivkontrolle. a: Integration des HIV-1 IIIB-Provirus in C8166-Zellen in Prozent. Die Behandlung mit dem Präimmunserum entsprach 100 %; alle Seren wurden in einer Verdünnung von 1:4 verwendet. b: Die Hemmung der Integration von HIV-1 IIIB durch das Ratten-Serum 71.2 war von der Dosis abhängig, wie anhand der Verdünnungsreihe des Serums gezeigt wurde. c: Die Neutralisation von HIV-1 IIIB wurde durch Immunoglobuline vermittelt, wie die IgG-Aufreinigung aus Präimmun- bzw. Immunserum zeigte. d, e: Hemmung der Integration des Provirus von HIV-1 Bal (c) und von HIV-1 SF162 (d) in Donor-Makrophagen nach der Behandlung mit den Ratten-Immunseren 71.2 und 79.3, die in einer Verdünnung von 1:8 eingesetzt wurden. Präimmunserum und mAb 2F5 wurden als Kontrollen verwendet.

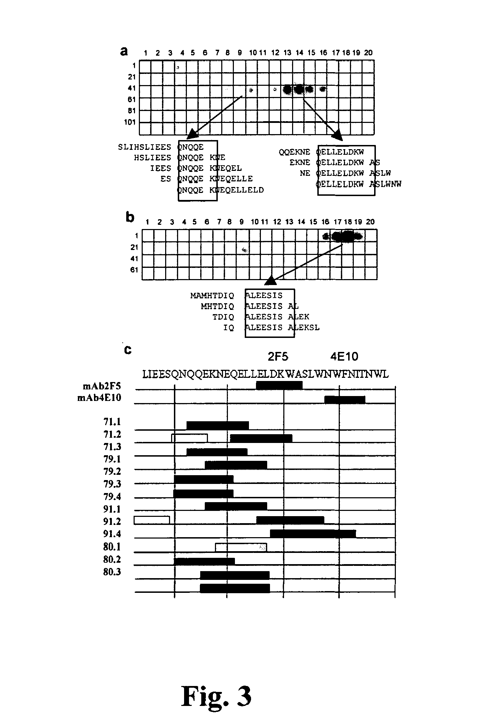

Fig. 3: Epitop-Mapping unter Verwendung von Pepspot-Membranen mit überlappenden Peptiden und von Ratten-Seren nach der Immunisierung mit dem Hybridprotein p15E/gp41. Für den Nachweis wurde die ECL-Methode verwendet. a, b: Ergebnisse mit dem neutralisierenden Ratten-Serum 71.2 unter Verwendung von überlappenden Peptiden, die dem gesamten gp41 aus HIV-1 (a) entsprachen, und von überlappenden Peptiden, die dem gesamtem p15E aus FeLV (b) entsprachen. Die Sequenzen der reagierenden Peptide sind dargestellt; die identifizierten Epitope sind eingerahmt. c, Zusammenfassung des Epitop-Mapping von allen Ratten-Antiseren unter Verwendung von Peptiden, die aus gp41 abgeleitet wurden, und Vergleich mit mAb 2F5 und mAb 4E10. Die Epitope wurden analog zu a und b identifiziert.

### BEISPIEL

Das Rückgrat von p15E wurde vom Vektor pCaln-p15E amplifiziert (aa476-512 und aa530-539) (Langhammer et al., Vaccine 23, 3341-3348 (2005)). Die DNA-Sequenzen der immunsuppressiven Domäne und der Peptide J und K (Nick et al., J. Gen. Virol. 71, 77-83 (1990)) wurden in einer zweistufigen PCR deletiert. Die DNA-Sequenz der E2-Region von HIV-1 IIIB gp41 wurde vom Vektor pNL4-3 amplifiziert (aa645-680). Beide amplifizierte Sequenzen wurden unter Verwendung der eingefügten Restriktionsstellen Notl, BamHI und EcoRI ligiert und in den Vektor pCal-n geklont (Stratagene). E. coli BL21 DE3-Zellen wurden transformiert und das mit dem 4 kDa Calmodulin-Bindungsprotein (CBP) fusionierte Hybridprotein wurde hergestellt (Fig. 1 a, b). Das Hybridprotein hatte eine Größe von 9,9 kDa, die von zusätzlichen 4 kDa des CBP am N-Terminus ergänzt wurde. Das p15E/gp41-Hybrid konnte im Western Blot mit mAb 2F5 nachgewiesen werden (Fig. 1 c), so dass das entsprechende Epitop im Hybrid vorhanden war. SDS-PAGE und Western Blot wurden nach Tacke et al., Xenotransplantation 8, 125-135 (2001) durchgeführt. 1 µg rekombinantes Hybrid p15E/gp41 wurden pro Spur eingesetzt.

Wistar-Ratten wurden von einem kommerziellen Anbieter bezogen und in Gruppen gehalten. 10 Ratten wurden zweimal (nach 0 bzw. 3 Wochen) intramuskulär mit 0,1 mg Hybridprotein p15E/gp41 in 0,5 ml gepufferter Salinlösung, die in 1,2 ml Montanide ISA 720 (Seppic, Frankreich, lot no.: 143521) emulgiert war, immunisiert (i.m.). Außerdem wurden 3 Ratten wie beschrieben immunisiert und einmal mit 0,1 mg Peptid, das von gp41 abgeleitet (aa656-680) und in 0,25 ml Montanide ISA 720 emulgiert war, wiederholt geimpft.

Die Neutralisationseffizienz der Seren wurde in Virus-Neutralisationsassays analysiert. Hierzu wurden HIV-1 IIIB in C8166-Zellen sowie die Primärisolate HIV-1 Bal und SF162 in frisch isolierten humanen Makrophagen verwendet. Der Virusstock für den Neutralisationsassay wurde als zellfreier Überstand von C8166-Zellen, die mit HIV-1 IIIB infiziert waren, hergestellt und auf nicht infizierten C8166-Zellen titriert. 50 µl zellfreier virushaltiger Überstand (1x10³ TCID₅₀) wurden verwendet, um 5x10⁴ C8166-Zellen, die in einem Well einer 96 U-Well-Mikrotiterplatte in einem Gesamtvolumen von 100 µl angesiedelt waren, zu infizieren. Präimmun- und Immunseren wurden bei 56°C für 30 min Hitze inaktiviert. Fortlaufende Verdünnungen der Seren wurden zum Virus gegeben, für 45 min bei 37°C inkubiert und auf die Zellen pipettiert. Nach einer Inkubation (37°C, 5% CO₂) von 72 h wurden die Zellen dreimal gefroren und aufgetaut und Lyse-Puffer mit 20 mg/ml Proteinase K in PCR-Puffer (50 mM KCl, 1,5 mM MgCl₂, 10 mM Tris-HCl, pH 8.4) hinzugefügt. Die Zellen wurden 3 h bei 56°C inkubiert. Anschließend wurde die Aktivität der Proteinase K 10 min bei 95°C gehemmt. Die Provirus-Integration in infizierte Zellen wurde mittels quantitativer Realtime-PCR wie folgt gemessen: 2 µl Zelllysat aus dem Neutralisationsassay wurden als Template in einer gp41-spezifischen Realtime-PCR mit den Primern SK68i 5'-GGARCAGCIGGAAGCACIATGG-3' und SK69i 5'-CCCCAGACIGTGAGITICAACA-3' sowie der Sonde 6Fam-TGACGCTGACGGTACAGGCCAGAC-dabcyl (Taqman Universal Master-Mix, Applied Biosystems) verwendet. Die PCR wurde unter Verwendung eines Stratagene MX4000 (55 Zyklen, Annealing-Temperatur: 55°C, keine Elongation) durchgeführt (Schweiger et al., J. Virol. Methods 67, 45-55 (1997)).

5 von 10 der immunisierten und wiederholt geimpften Ratten zeigten eine HIV-1 IIIB-Neutralisation von bis zu 99 % (Fig. 2a). Die Titer der neutralisierenden Antikörper betrugen bis zu 1:16 (Fig. 2b).

Für Neutralisationsassays mit Immunoglobulinen wurden Protein G-Säulen (Montage, Millipore) zum Reinigen von IgG verwendet. Aufgereinigte IgG aus dem Immunserum 91.2 zeigten eine Neutralisation von bis zu 80 % im Vergleich mit den gereinigten IgG aus dem Präimmunserum. Die Neutralisation von HIV-1 IIIB war folglich durch Immunoglobuline vermittelt (Fig. 2c).

Die Neutralisationsassays mit den HIV-1-Isolaten Bal und SF162 wurden auf differenzierten Makrophagen durchgeführt (Ruppach et al., J. Virol. 74, 5403-5411 (2000)) und ― wie oben beschrieben ― analysiert. Zwei Antiseren (Ratten 71.2, 79.3) neutralisierten bis zu 81 % bei einer Verdünnung des Serums von 1:8 (Fig. 2d, e). Dagegen zeigten die Antiseren aus Tieren, deren Auffrischungsimpfung das vom gp41-abgeleitete Peptid erhielt, keinen neutralisierenden Effekt, so dass die Auffrischungsimmunisierung mit dem Hybridprotein die bessere Strategie darstellte.

Anschließend wurden die Rattenseren im ELISA analysiert. 96 Wellplatten wurden mit 500 ng/Well Peptid mit dem 2F5/4E10-Epitop, abgeleitet von gp41 (aa656-680), bedeckt und die Präimmun- und Immunseren wurden in Verdünnungsreihen eingesetzt. Der ELISA wurde nach Langhammer et al., Vaccine 23, 3341-3348 (2005) durchgeführt. Die Titer der bindenden Antikörper reichten von 1:400 bis 1:6400 und bestätigten die Induktion gp41-spezifischer Antikörper.

Um die von den neutralisierenden Antiseren erkannten Epitope zu identifizieren, wurde ein Epitop-Mapping unter Verwendung von Pepspot-Membranen mit überlappenden Peptiden durchgeführt (Fig. 3a, b). Pepspot-Bibliotheken des gesamten gp41 aus HIV-1 IIIB und der p15E-Ectodomäne aus dem FeLV-A Glasgow-Stamm wurden verwendet (15-mer Peptide, die bis zu 13 Aminosäuren überlappten, adsorbiert auf CelluloseMembranen, Jerini Biotools). Das experimentelle Vorgehen orientierte sich an Fiebig et al., Virology 307, 406-413 (2003) und Langhammer et al., Vaccine 23, 3341-3348 (2005). Für gp41 wurden Epitope (SEQ ID NOs: 68 und 69) gefunden, die in unmittelbarer Nähe zum 2F5-Epitop ELDKWA lokalisiert sind. Im Fall der Ratte 71.2 überlappte das gefundene Epitop (SEQ ID NO: 70) teilweise mit dem 2F5-Epitop. Die Aminosäuresequenzen der SEQ ID NOs: 68 bis 70 sind auch in der neuen Epitop-Domäne von gp41 mit der Aminosäuresequenz der SEQ ID NO: 67 enthalten, die durch p15E/gp41-induzierte Antiseren erkannt wird. Bei der Ratte 91.2 war sowohl eine Überlappung des ermittelten Epitops der Aminosäuresequenz der SEQ ID NO: 71 mit dem 2F5-Epitop als auch mit dem 4E10-Epitop festzustellen (Fig. 3c). Die in den Seren enthaltenen Antikörper erkannten immer eine N-terminale und eine C-terminale Epitop-Region, die sich nach der Wechselwirkung beider Helices im Zuge intramolekularer Konformationsveränderungen in unmittelbarer Nähe befinden. Darüber hinaus wurde auch im p15E-Rückgrat ein Epitop gefunden (SEQ ID NO: 90), das als E1b beschrieben ist (Fig. 3b).

## Patentansprüche

1. Immunogenes Konstrukt umfassend Aminosäuresequenzen aus zwei retroviralen transmembranen Hüllproteinen, **dadurch gekennzeichnet, dass** das immunogene Konstrukt ein Hüllprotein eines ersten Retrovirus oder einen Teil davon umfasst, wobei das Hüllprotein oder dessen Teil in einem Bereich zwischen Fusionsdomäne und N-terminaler helikaler Region (NHR) eine erste Aminosäuresequenz umfasst, und in einem Bereich zwischen Membrandurchgang und C-terminaler helikaler Region (CHR) eine zweite Aminosäuresequenz beinhaltet, wobei die zweite Aminosäuresequenz eine Sequenz aus einem Hüllprotein eines zweiten Retrovirus ist und diese zweite Aminosäuresequenz die zu ihr in der Sekundär- und/oder Tertiärstruktur homologe zweite Aminosäuresequenz des Hüllproteins des ersten Retrovirus ersetzt, wobei die erste und die zweite Aminosäuresequenz des Konstruktes zueinander antiparallel angeordnet sind,
und/oder das Konstrukt, das die die jeweiligen Aminosäuresequenzen codierende DNA umfasst.

2. Immunogenes Konstrukt nach Anspruch 1, **dadurch gekennzeichnet, dass** das Hüllprotein des ersten und zweiten Retrovirus ausgewählt ist aus der Gruppe gp41, p15E, GP, gp20, gp21, gp30, gp36, gp37, gp40, gp45 und gp160.

3. Immunogenes Konstrukt nach Anspruch 1 oder 2, **dadurch gekennzeichnet, dass** das Hüllprotein des ersten Retrovirus ein Hüllprotein eines Gammaretrovirus ist, vorzugsweise p15E aus FeLV.

4. Immunogenes Konstrukt nach einem der Ansprüche 1 bis 3, **dadurch gekennzeichnet, dass** das Hüllprotein des zweiten Retrovirus ein Hüllprotein eines Lentivirus ist, vorzugsweise gp41 aus HIV-1.

5. Immunogenes Konstrukt nach einem der Ansprüche 1 bis 4, **dadurch gekennzeichnet, dass** die erste Aminosäuresequenz ein Bereich des Hüllproteins zwischen Fusionsdomäne und Cystein-Cystein-Schleife ist, vorzugsweise eine Aminosäuresequenz der SEQ ID NOs: 1 bis 5, 7 bis 12, 24, 26, 28, 30, 32, 34, 36, 38, 40, 42, 44, 46, 48, 50, 52, 54, 56, 58, 60, 62, 64, 72 bis 82, 87 und 89, oder sie codierende DNA, oder Varianten, Mutanten, Teile der Aminosäuresequenzen oder zu 80 % homologe Sequenzen, die die gleichen Eigenschaften besitzen.

6. Immunogenes Konstrukt nach einem der Ansprüche 1 bis 5, **dadurch gekennzeichnet, dass** die zweite Aminosäuresequenz ausgewählt ist aus der Gruppe der Aminosäuresequenzen der SEQ ID NOs: 6, 13 bis 23, 25, 27, 29, 31, 33, 35, 37, 39, 41, 43, 45, 47, 49, 51, 53, 55, 57, 59, 61, 63, 65, 66 bis 71, 83 bis 86 und 88, oder sie codierender DNA, oder Varianten, Mutanten, Teile der Aminosäuresequenzen oder zu 80 % homologe Sequenzen, die die gleichen Eigenschaften besitzen.

7. Immunogenes Konstrukt nach Anspruch 5 und 6, **dadurch gekennzeichnet, dass** die erste Aminosäuresequenz eine Aminosäuresequenz der SEQ ID NOs: 58, 78 und 79 ist und die zweite Aminosäuresequenz ausgewählt ist aus der Gruppe der Aminosäuresequenzen der SEQ ID NOs: 6, 13 bis 23, 66 bis 71 und 83 bis 85.

8. Immunogenes Konstrukt nach Anspruch 7, **dadurch gekennzeichnet, dass** die erste Aminosäuresequenz die Aminosäuresequenz der SEQ ID NO: 78 und die zweite Aminosäuresequenz die Aminosäuresequenz der SEQ ID NO: 66 ist.

9. Immunogenes Konstrukt nach Anspruch 1, **dadurch gekennzeichnet, dass** in der N-terminalen helikalen Region die immunsuppressive Domäne isu und das Peptid J deletiert sind und in der C-terminalen helikalen Region das Peptid K deletiert ist.

10. Pharmazeutisches Mittel, umfassend mindestens eines der immunogenen Konstrukte nach einem der Ansprüche 1 bis 9, vorzugsweise zusammen mit pharmazeutisch verträglichen Hilfsstoffen.

11. Pharmazeutisches Mittel nach Anspruch 10 zur prophylaktischen oder therapeutischen Behandlung von retroviralen Erkrankungen, vorzugsweise retroviralen Immunerkrankungen.

12. Pharmazeutisches Mittel nach Anspruch 11 zur prophylaktischen oder therapeutischen Behandlung einer HIV-Infektion.

13. Pharmazeutisches Mittel nach einem der Ansprüche 10 bis 12 zur oralen, transmucosalen, rektalen, intravenösen, subkutanen und/oder intramuskulären Anwendung.

14. Pharmazeutisches Mittel nach einem der Ansprüche 10 bis 13, **dadurch gekennzeichnet, dass** es als Tablette, Kapsel, Pulver, Lösung, Dispersion oder Suspension vorliegt.

15. Neutralisierende Antikörper, hergestellt durch Immunisierung mit dem immunogenen Konstrukt nach einem der Ansprüche 1 bis 9.

16. Verwendung der neutralisierenden Antikörper nach Anspruch 15 zur Diagnose, Prophylaxe, Therapie und/oder Verlaufskontrolle von retroviralen Erkrankungen, vorzugsweise HIV.

17. Verwendung des immunogenen Konstruktes nach einem der Ansprüche 1 bis 9 zur Diagnose, Prophylaxe, Therapie und/oder Verlaufskontrolle von retroviralen Erkrankungen, vorzugsweise HIV.

18. Immunologisches Verfahren für den Nachweis von HIV-1-, FeLV-, BIV-, CEAV-, EIAV1-, FIV-, OMVV-, SIVmac-, SIVcpz-, VILV-, HIV-2-, RSV-, ALV-, JSRV-, SRV-, GALV-, MLV-, BLV-, HTLV-1-, HTLV-2- und/oder KoRV-Antikörpern in einer biologischen Probe, umfassend
a) Beschichten einer festen Phase mit dem immunogenen Konstrukt nach einem der Ansprüche 1 bis 9,
b) Inkubieren der festen Phase mit der biologischen Probe,
c) Inkubieren der festen Phase mit einem anti-humanen Antikörper, der die Klassen IgA, IgM, IgG nachweisen kann und der mit einer nachweisbaren Kennzeichnung markiert ist, und
d) Nachweis der Kennzeichnung zur Bestimmung der Anwesenheit bindender Antikörper gegen die genannten Retroviren in der Probe.

19. Immunologisches Verfahren für den Nachweis von retroviralen Antigenen in einer biologischen Probe, umfassend
a) Beschichten einer festen Phase mit mindestens einem neutralisierenden Antikörper nach Anspruch 15,
b) Inkubieren der festen Phase mit der biologischen Probe,
c) Inkubieren der festen Phase mit einem vom ersten unterschiedlichen zweiten Antikörper gegen die gesuchten retroviralen Antigene, wobei der zweite Antikörper in einem Organismus nach Immunisierung mit dem immunogenen Konstrukt nach einem der Ansprüche 1 bis 9 gewonnen wurde, und
d) Nachweis des gekoppelten zweiten Antikörpers zur Bestimmung der Anwesenheit des Antigens in der Probe.

20. Verfahren zur Induktion einer Antikörperantwort, **dadurch gekennzeichnet, dass** das immunogene Konstrukt nach einem der Ansprüche 1 bis 9 einem Organismus appliziert und die Bildung neutralisierender Antikörper induziert wird.

21. Verfahren zur passiven Immunisierung eines Organismus, **dadurch gekennzeichnet, dass** die nach dem Verfahren nach Anspruch 20 induzierten Antikörper gewonnen und einem Organismus appliziert werden.
